# EUROPEAN PATENT SPECIFICATION

(11) **EP 0 166 356 B1**
(45) Date of publication and mention of the grant of the patent: **01.09.1993**
(21) Application number: 85107501.0
(22) Date of filing: 18.06.1985
(51) Int. Cl.: C07D 403/12, C07D 409/12, C07D 243/16, A61K 31/55

(54) **2-Substituted-aminomethyl-1,4-benzodiazepines, process for their preparation and pharmaceutical compositions containing them**
2-Substituierte Aminomethyl-1,4-benzodiazepine, Verfahren zu ihrer Herstellung und sie enthaltende pharmazeutische Zusammensetzungen
Aminométhyl-1,4-benzodiazépines substituées sur la position 2, procédé pour leur préparation et compositions pharmaceutiques les contenant

(30) Priority: 26.06.1984 US 624842
(43) Date of publication of application: 02.01.1986
(73) Proprietor: Merck & Co., Inc., Rahway New Jersey 07065-0900 (US)
(72) Inventor: Bock, Mark G., Hatfield, PA 19440 (US); Freidinger, Roger M., Hatfield, PA 19440 (US)
(74) Representative: Blum, Rudolf Emil Ernst

(56) References cited:
- EP-A- 0 031 080
- EP-A- 0 054 839
- DE-A- 2 353 187
- FR-A- 2 248 278
- US-A- 4 307 237
- CHEMICAL ABSTRACTS, vol. 85, no. 9, 30th August 1976, page 62, abstract no. 56967t, Columbus, Ohio, US; J.H.B. SAUNDERS et al.: "Effect of diazepam and hyoscine butylbromide on response to secretin and cholecystokinin-pancreozymin in man"
- NATURE, vol. 312, 22nd November 1984, pages 363-364, Basingstoke, GB; J. BRADWEJN et al.: "Benzodiazepines antagonize cholecystokinin-induced activation of rat hippocampal neurones"

## Description

The present invention is directed to novel 2-substituted-aminomethyl-1,4-benzodiazepines which have been found to be antagonists of the function of cholecystokinins (CCK), to the preparation of these compounds, and to pharmaceutical compositions containing these compounds as active ingredient for antagonizing the function of CCK, which antagonism is useful, e.g., for the treatment and prevention of disorders of the gastrointestinal, central nervous and appetite regulatory systems of mammals, especially of humans.

### BACKGROUND OF THE INVENTION

Cholecystokinins (CCK) are neuropeptides [see Mutt and Jorpes, Biochem. J., 125, 678 (1971)] which exist in both gastrointestinal tissue and the central nervous system (V. Mutt, Gastrointestinal Hormones, G. B. J. Glass, ed., Raven Press, N.Y., 1980, p. 169), and include e.g., CCK-33, a neuropeptide of thirty-three aminoacids and its carboxylterminal octapeptide, CCK-8. These molecules are believed to be physiological satiety hormones and, therefore, may play an important role in appetite regulation (G. P. Smith, Eating and Its Disorders, A. J. Stunkard and E. Stellar, Eds., Raven Press, New York, 1984, p. 67).

In addition, CCK's stimulate colonic motility, gall bladder contraction; and pancreatic enzyme secretion, and inhibit gastric emptying. CCK's reportedly also co-exist with dopamine in certain mid-brain neurons, and thus may additionally play a role in the functioning of dopaminergic systems in the brain, as well as serve as neurotransmitters in their own right. See: A. J. Prange et al., "Peptides in the Central Nervous System", Ann. Repts. Med. Chem., 17, 31, 33 (1982), and references cited therein; J. A. Williams, Biomed. Res., 3, 107 (1982); and J. E. Morley, Life Sci., 30, 479 (1982).

### DESCRIPTION OF THE PRIOR ART

Antagonists of CCK are useful for preventing or treating CCK-related disorders of the gastrointestinal, central nervous and appetite regulatory systems of mammals, specially of humans.

Three distinct chemical classes of CCK-receptor antagonists have been reported.

The first class comprises derivatives of cyclic nucleotides, of which dibutyryl cyclic GMP has been shown to be the most potent by detailed structure-funcion studies (see N. Barlos et al., Am. J. Physio., 242, G161 (1982) and P. Robberecht et al., Mol. Pharmacol., 17, 268 (1980)).

The second class comprises peptide antagonists which are C-terminal fragments and analogs of CCK, of which both shorter (Boc-Met-Asp-Phe-NH₂, Met-Asp-Phe-NH₂) and longer (Cbz-Tyr(SO₃H)-Met-Gly-Trp-Met-Asp-NH₂) C-terminal fragments of CCK can function as CCK antagonists, according to recent structure-function studies (see R.T. Jensen et al., Biochim. Biophys. Acta., 757, 250 (2983), and M. Spanarkel et al., J. Biol. Chem., 258, 6746 (1983)).

The third class of CCK receptor antagonists comprises the amino acid derivatives: proglumide, a derivative of glutamaric acid, and the N-acyl tryptophans, including para-chlorobenzoyl-L-tryptophan (benzotript), (see W.F. Hahne et al., Proc. Natl. Acad.Sci. U.S.A., 78, 6304 (1981) and R.T. Jensen et al., Biochim. Biophys. Acta, 761. 269 (1983).

All of these compounds, however, are relatively weak antagonists of CCK (IC₅₀: generally 10⁻⁴M, but down to 10⁻⁶M in the case of the peptides). The peptide CCK-antagonista have furthermore substantial stability and absorption problems.

In the publication of J. Bradwein et al., Nature, 312, 363 (1984) which is an intermediate publication three benzodiazepine derivatives were investigated as to their activity of antagonizing CCK in the brain, i.e. antagonizing the CCK-induced activation of rat hippocampal neurones. Two of the three tested benzodiazepines correspond to the following formula A 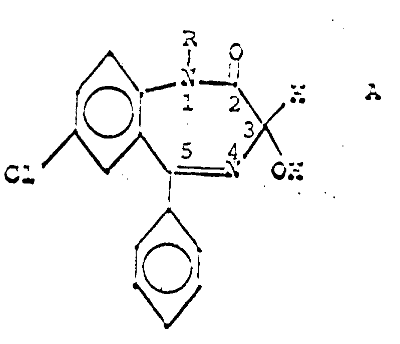
and in the corresponding benzodiazepine which is named lorazepam the radical R is hydrogen and in the corresponding compound named flurazepam the radical R is a group of formula

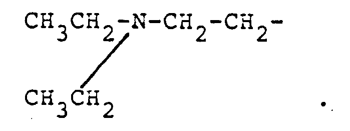

In both said compounds the benzene nucleus which is bonded to the carbon atom in the position 5 is substituted in its ortho position with a chlorine atom. The third tested compound which is named diazepam differs from the compounds of formula A in that to the carbon atom in the position 3 of the seven-membered ring there are bonded two hydrogen atoms and no hydroxy group and in said compound the radical R is methyl and the benzene nucleus bonded to the carbon atom in the position 5 is unsubstituted.

In said benzodiazepine derivatives accordingly the carbon atom in the position 2 is a carbonyl group and the carbon atom in the position 3 is a group of formula H-C-OH or -CH₂-.

The benzodiazepine (BZD) structure class has been widely exploited as therapeutic agents, specially as central nervous system (CNS) drugs. These compounds exhibit strong binding to "benzodiazepine receptors" in vitro, but have not been reported to bind to CCK or gastrin receptors.

In the German Offenlegungsschrift 2 353 187 there are described benzodiasepine derivatives in which to the carbon atom in the position 2 of the seven-membered ring there is bonded an amino substituted methyl group, the carbon atom in the position 3 is a -CH₂-group and to the carbon atom in the position 5 there is bonded an optionally substituted phenyl nucleus. Said benzodiazepines accordingly correspond to the following formula B 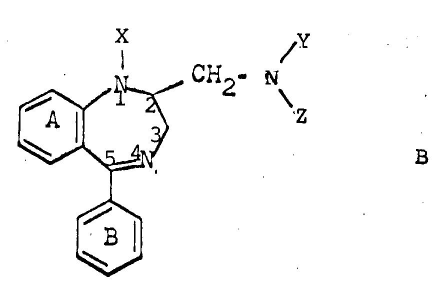
in which
- X: is hydrogen, lower alkyl, alkenyl, cycloalkyl or cycloalkenyl, which radicals optionally can be substituted and
the group of formula 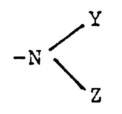
is either an NH₂ group which is acylated with an aliphatic or aromatic dicarboxylic acid forming a cyclic structure or in said group
- Y: is hydrogen, phenyl, lower alkyl, alkenyl, cycloalkyl or cycloalkenyl, which radicals optionally can be substituted and
- Z: is a group having the structure

-CO-Y

-COOY

or

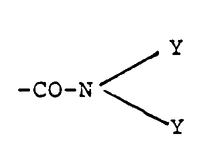

wherein Y is as defined above and
the phenyl nuclei A and B can be optionally substituted with substituents selected from the group comprising nitro, halo, trifluoro, methyl, loweralkyl, loweralkoxy methylenedioxy or ethylenedioxy respectively (see claim 1).

There is stated on page 2, second paragraph, of said publication that the corresponding substances can be used for influencing the central nervous system and they specially have an anticonvulsant, sedatic, a tranquilizing and a musclerelaxant activity.

In the U.S. patent 4 307 237 there are described compounds which have a benzodiazepine structure, provided that the ring corresponding to the ring A of the benzodiazepines of formula B is an optionally substituted benzene nucleus and not one of the five-membered heterocycles which are as well disclosed in said patent. The substituent bonded to the carbon atom in the position 5 of the benzodiazepine structure is an optionally substituted phenyl or pyridyl nucleus. The compounds described in the abstract and in column 1 of said patent as well as the preferred compounds of formulae Ia, Ib, Ic and Id disclosed in columns 3, 5 and 6 of said patent, however, differ from the benzodiazepines having the above stated formula B in that the substituent X and the substituents Y and Z form together with the nitrogen atom to which they are bonded a five-membered heterocyclic ring, i.e. an imidazo structure which is condensed with the seven-membered ring of the benzodiazepine. Said compounds having totally three condensed rings are stated to have the pharmaceutical activity of anticonvulsants, musclerelaxants, anxiolytic and sedative agents and also corresponding compounds which result from a cleaving of the seven-membered ring between the carbon atom in the position 5 and the nitrogen atom in the position 4 are stated to have a pharmacological activity (see column 7).

Structurally related to the benzodiazepine compounds of the above stated formula B, however, are those starting materials having the formulae V and VI disclosed in columns 9 and 10 of said U.S. patent in which to the carbon atom in the position 2 of the seven-membered ring there is bonded a methyl group which is substituted with an amino group NH₂ or a corresponding amino group which is acylated with an aliphatic carboxylic acid or a carboxylic acid in which the group COOH is bonded to an optionally substituted phenyl or pyridyl nucleus.

The substituent corresponding to the group X of the benzodiazepines having the formula B stated above can be in said starting materials either a hydrogen atom or an acyl group derived from one of the above named carboxylic acids. There however is nowhere stated in said U.S. patent that the starting materials disclosed therein have any pharmaceutical activity.

In the French patent publication 2 248 278 there are described benzodiazepines which can be as well illustrated with the above stated formula B, provided that the methyl group, bonded to the carbon atom in the seven-membered ring is substituted with such a radical R₂ which has the meaning of an amino group or an acylated amino group. In said compound the group corresponding to the group 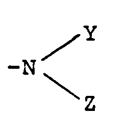
of the compounds of' formula B can be a corresponding group in which the radicals Y and Z are hydrogen, alkyl, aralkyl or aryloxy alkyl or they can form together with the nitrogen atom to which they are bonded a heterocyclic structure (see page 1, lines 21 - 34 of said publication).

The corresponding compounds are stated to have an anticonvulsant, tranquilizing, sedatic and musclerelaxant activity (see page 2, lines 10 - 16).

Also in the German patent specification 2 221 558 there are described benzodiazepine derivatives which can be illustrated with the above stated formula B provided that the methyl group which is bonded to the carbon atom in the position 2 of the benzodiazepine nucleus is substituted with a radical R₄ which has the meaning of an amino group. In the compounds disclosed in said patent the radical which corresponds to the group X of the compounds of formula B has to be a hydrogen atom or a methyl group and the benzene nuclei A and B are unsubstituted or substituted with substituents selected from the group comprising halo atoms, nitro groups and trifluoro methyl groups. The corresponding compounds have an influence onto the central nervous system and they have an anticonvulsant, sedatic, tranquilizing and musclerelaxant activity (see column 4, lines 1 - 5).

In the European patent publication 0 054 839 there are disclosed benzodiazepine derivatives which can be illustrated with the above stated formula B. In said compounds the radical corresponding to the group X is hydrogen, loweralkyl, loweralkenyl or cyclopropylmethyl and the amino group bonded to the methyl group in the position 2 is an acylated amino group in which the radical corresponding to the group Y of the compounds of formula B is hydrogen, loweralkyl or loweralkenyl and the group corresponding to the group Z is a group of formula 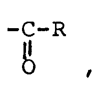
wherein R is an optionally substituted phenyl nucleus which is directly bonded or bonded via a methylene or ethylene group. Said compounds are stated to have a strong analgetic activity and besides said activity also a sedatic activity, a diuretic activity and an antiarrhythmic activity (see page 1, last paragraph).

In the European patent publication 0 031 080 there are as well described benzodiazepine derivatives which can be illustrated with the above stated formula B. The substituent corresponding to the group X is hydrogen, a loweralkyl or alkenyl group or a cyclopropyl methyl group. Furthermore the radical corresponding to the substituent Y is a loweralkyl or alkenyl group and the radical corresponding to the group
- Z: is an acyl group of formula

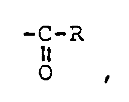

wherein the radical R is a five-membered or six-membered heterocycle which is selected from the group comprising a furan nucleus, a thiophene nucleus, a pyrrol nucleus, an N-loweralkyl pyrrol nucleus or a pyridine nucleus and wherein said pyrrol or N-alkyl pyrrol nucleus can be substituted with alkyl having 1 - 4 carbon atoms and furan nuclei and thiophene nuclei can be further substituted with chloro, bromo, lower-alkyl, loweralkoxy or nitro.

There is stated on page 2, first paragraph of said patent that the corresponding benzodiazepine compounds have in addition to their activity as psychopharmacon and their diuretic and antiarrythmic activity also a strong analgetic activity and only low toxic properties.

In the U.S. patent 4 325 957 there are as well disclosed certain 2-acylaminomethyl-1H-2,3-dihydro-1,4-benzodiazepine derivatives. However, those derivatives were suggested as simply possessing strong analgesic activities, in addition to psychopharmacological, diuretic and antiarrythmic properties.

Additonally, the compound corresponding to formula B stated before wherein the benzene nucleus A is unsubstituted, the benzene nucleus B is substituted in its position 3 with a fluoro atom,
- X: is methyl,
- Y: is hydrogen and
- Z: is an acyl group of formula

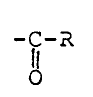

wherein R is a thiophene nucleus bonded to the position 3 has been reported to be an opiate-agonist with selectivity for the kappa receptor [see D. Roemer et al., Nature, 298, 759 (1982)] and its (-)-enantiomer is preferred for high analgesic activity [see H. Kley et al., Eur. J. Pharmacol., 87, 503 (1983)]. This compound has also been shown to increase food intake in rats [see J.E. Morley et al., Euro. J. Pharmacol., 93, 265 (1983)].

Accordingly none of the benzodiazepine derivatives disclosed in the prior art have been previously reported to antagonize the function of cholecystokinins, specially the excessive function of cholecystokinins in disease state.

It was an aim of the present invention to develop new benzodiazepine derivatives which are able to antagonize cholecystokinins in the brain as well as also in the gut.

### DESCRIPTION OF THE PRESENT INVENTION

One object of the present invention are 2-substituted-aminomethyl-1,4-benzodiazepines which have the formulae I, II or V 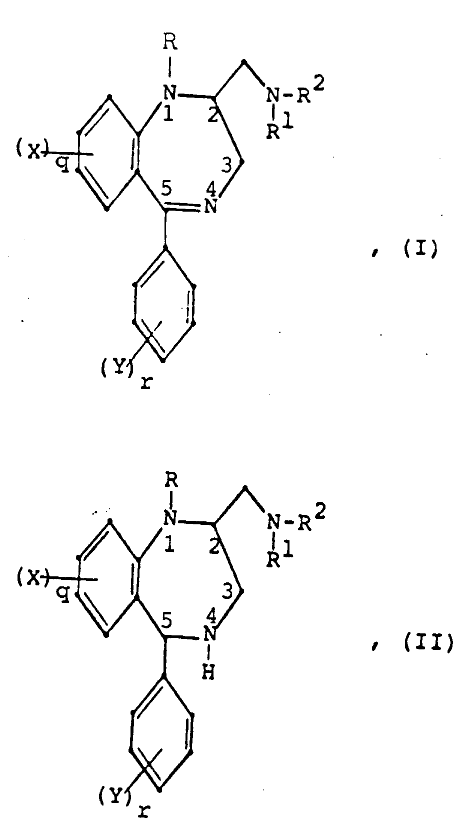 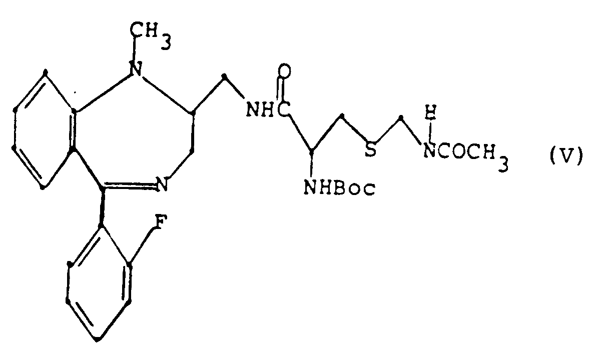
in which formulae
- X: is fluoro, chloro, bromo, alkyl having 1 - 4 carbon atoms, alkoxy having 1 - 4 carbon atoms, alkylthio having 1 - 4 carbon atoms, hydroxy, nitro, cyano, amino or trifluoro-methyl and the corresponding substituents X are attached at the position 7 and/or at the position 8;
- q: is zero, 1 or 2,
- Y: has the same meaning as X and the corresponding substituent may be attached at any of the positions 2 - 6 on the aromatic ring,
- r: is 1 or 2,
- R: is hydrogen, alkyl having 1 - 4 carbon atoms, alkenyl having up to 4 carbon atoms, cycloalkyl having 3 - 5 carbon atoms or acetyl,
- R¹: is hydrogen, alkyl having 1 - 4 carbon atoms or cycloalkyl having 3 - 5 carbon atoms,
- R²: is either a substituted alkyl group of formula 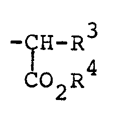 wherein
- R³: is a group having the formulae
- (CH₂)ₙ-C₁-C₄-alkyl,
- (CH₂)ₙ-2-indole,
- (CH₂)ₙ-3-indole, or
- (CH₂)ₙ-phenyl
   in which
   n is 0 - 4 and
   the phenyl is unsubstituted or mono- or disubstituted with substituents X
   which are as defined above and
- R⁴: is hydrogen or alkyl having 1 - 4 carbon atoms or
the radical
- R²: is an acyl group having the formula 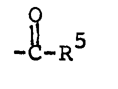 wherein the radical
- R⁵: is one of the following groups:
a) 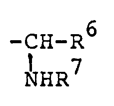
wherein
- R⁶: is (CH₂)ₙ-2-indole or
(CH₂)ₙ-3-indole, in which group n is 0 - 4 and
- R⁷: is hydrogen or a group of formulae COOR⁸, or 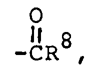 in which
- R⁸: is an alkyl group having 1 - 4 carbon atoms,
b) -(CH₂)ₘSCH₂NHCOCH₃
wherein
- m: is 1 - 4
c) 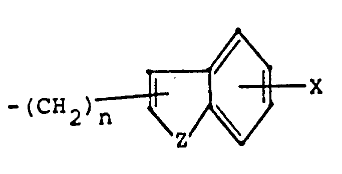
wherein
- Z: is O, S or NR,
wherein
- R: is as defined above,
the group -(CH₂)ₙ- is attached at the 2- or 3-position,
- n: is 0 - 4 and
- X: is as defined above,
d) 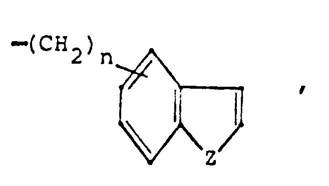
wherein
the group -(CH₂)ₙ- is attached at the 4- or 5-position and
Z and n are as defined in (c) above;
e) -(CH₂)ₘCO₂CH₂phenyl,
wherein
- m: is 1 - 4,
f) -CHOHC₆H₅;
g) 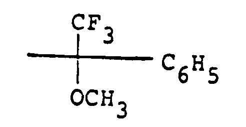 or
h) pyrazine, which is unsubstituted or monosubstituted, where the substituents may be Cl, COOR⁸, CN or NO₂, wherein R⁸ is as defined in a) above;
and optical isomers of the compounds of formula I and salts or quaternary ammonium salts of the compounds of formulae I or II.

Preferred inventive compounds of formulae I or II are those in which
- X: is fluoro or chloro,
- Y: has the same meaning as X,
- q and r: are as defined above,
- R: is hydrogen or alkyl having 1 - 4 carbon atoms,
- R¹: is hydrogen
- R²: is either a substituted alkyl group of formula 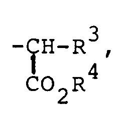 wherein
- R³: is a group having the formulae
- (CH₂)ₙ-2-indole,
- (CH₂)ₙ-3-indole, or
- (CH₂)ₙ-phenyl
in which
- n: is 1 and
the phenyl is unsubstituted or mono- or disubstituted with substituents X which are as defined above and
- R⁴: is alkyl having 1 - 4 carbon atoms or the radical
- R²: is an acyl group having the formula 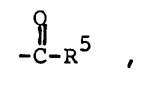 wherein the radical
- R⁵: is one of the following groups:
a) 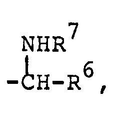
wherein
- R⁶: is -CH₂-2-indole or -CH₂-3-indole and
- R⁷: is as defined before,
c) 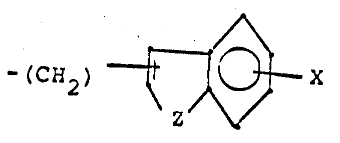
wherein Z and X are as defined before,
d) 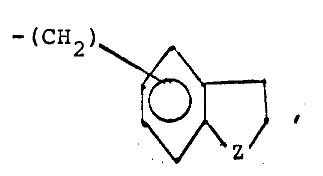
wherein
- Z: is as defined above in (c) and the group -CH₂- is attached at the 4- or 5-position or
f) -CHOHC₆H₅.

Particularly preferred compounds of formula I are the following substances:
1-methyl-2-(2'-indolecarbonyl)aminomethyl-5-(2'-fluorophenyl)-2,3-dihydro-1H-1,4-benzodiazepine,
1-methyl-2-(4-thianaphthenemethylcarbonyl)aminomethyl-5-(2'-fluorophenyl)-2,3-dihydro-1H-1,4-benzodiazepine,
1-methyl-1-(2-L-hydroxy-2-phenylacetyl)aminomethyl-5-(2'-fluorophenyl)-2,3-dihydro-1H-1,4-benzodiazepine,
1-methyl-2-(1H-indol-3-yl)methylcarbonylaminomethyl-5-(2'-fluorophenyl)-2,3-dihydro-1H-1,4-benzodiazepine,
1-methyl-2-[1-(S)-1-methoxycarbonyl-2-phenylethylamino] methyl-5-(2'-fluorophenyl)-2,3-dihydro-1H-1,4-benzodiazepine,
1-methyl-2-[2-((1,1-dimethylethoxy)carbonyl)amino-3-(1H-indol-3-yl)propanoyl]aminomethyl-5-(2'-fluorophenyl)-2,3-dihydro-1H-1,4-benzodiazepine, 1-methyl-2-[2-amino-3-(1H-indol-3-yl)propanoyl]aminomethyl-5-(2'-fluorophenyl)-2,3-dihydro-1H-1,4-benzodiazepine, 1-methyl-2-(2-methoxy-2-trifluoromethyl-2-phenylacetyl)aminomethyl-5-(2'-fluorophenyl)-2,3-dihydro-1H-1,4-benzodiazepine, 1-methyl-2-[2-(S)-((1,1-dimethylethoxy)carbonyl)amino-3-acetamidomethylmercaptopropanoyl]aminomethyl-5-(2'-fluorophenyl)-2,3-dihydro-1H-1,4-benzodiazepine, 1-methyl-2-benzylsuccinoyl-aminomethyl-5-(2'-fluorophenyl)-2,3-dihydro-1H-1,4-benzodiazepine, and 1-methyl-2-(acetamidomethylmercaptoacetyl)aminomethyl-5-(2'-fluorophenyl)-2,3-dihydro-1H-1,4-benzodiazepine.

Particularly preferred compounds of formula II include 1-methyl-2-(2'-indolecarbonyl)aminomethyl-5-(2'-fluorophettyl)-2,3,4,5-tetrahydro-1H-1,4-benzodiazepine, 1-methyl-2-(4-thianaphthenemethylcarbonyl)aminomethyl-5-(2'-fluorophenyl)-2,3,4,5-tetrahydro-1H-1,4-benzodiazepine, 1-methyl-2-(2-L-hydroxy-2-phenylacetyl)aminomethyl-5-(2'-fluorophenyl)-2,3,4,5-tetrahydro-1H-1,4-benzodiazepine, 1-methyl-2-(1H-indol-3-yl)methylcarbonylaminomethyl-5-(2'-fluorophenyl)-2,3,4,5-tetrahydro-1H-1,4-benzodiazepine,

The pharmaceutically-acceptable salts of the compounds of the instant invention include the conventional non-toxic salts or the quaternary ammonium salts of the compounds of this invention formed, for example, from non-toxic inorganic or organic acids. For example, such conventional non-toxic salts include those derived from inorganic acids, such as hydrochloric, hydrobromic, sulfuric, sulfamic, phosphoric or nitric acid; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, theophylline, 8-chlorotheophylline, p-aminobenzoic, p-acetamidobenzoic, methanesulfonic, ethane disulfonic, oxalic, or isethionic acid.

A further object of the present invention is a process for preparing 2-substituted aminomethyl-1,4-benzodiazepines having the formula I 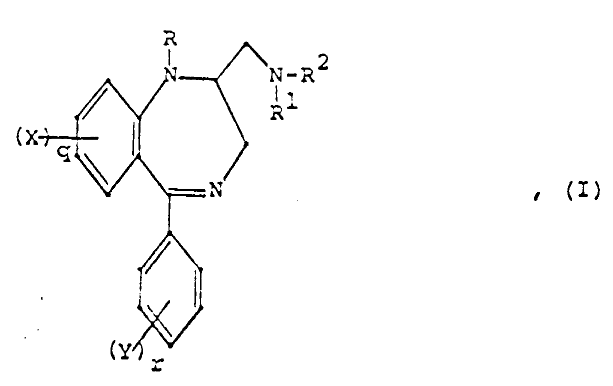
wherein
X, Y, q, r, R, R¹ and R² are as defined in connection with the compounds of formulae I and II.

Said process is characterized in that a compound of formula III 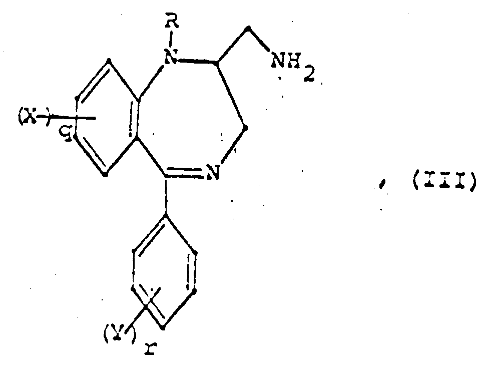
or an acid addition salt of the compound of formula III,
in which formula III X, Y, q, r and R are as defined in formula I,
is acylated with a carboxylic acid having the formula R⁵-COOH,
wherein
- R⁵: is as defined in formula I,
or a derivative of said carboxylic acid,
to yield compounds of formula Ia 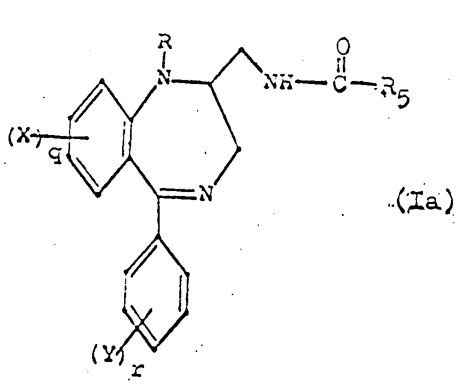
which compounds of formula Ia are optionally alkylated by substituting the hydrogen atom bonded to the nitrogen atom of the acid amide group by an alkyl group having 1 - 4 carbon atoms or a cycloalkyl group having 3 - 5 carbon atoms or in that
the amino compound of formula III stated before is alkylated with a compound of formula 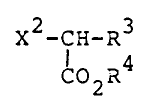
wherein X² is chloro, bromo or iodo or the group

-O-SO₂-R

wherein R is alkyl having 1 - 4 carbon atoms or optionally substituted phenyl yielding the corresponding compounds of formula Ib 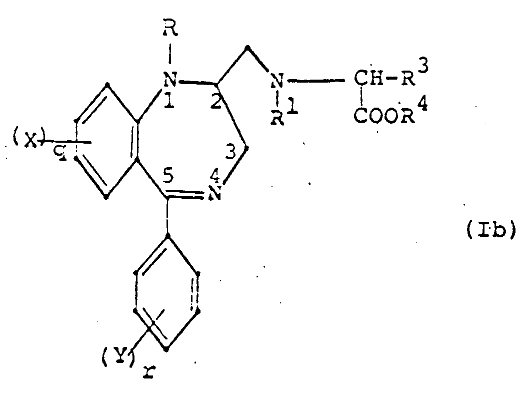
wherein R³ and R⁴ are as defined before in connection with formula I.

According to a further embodiment the compounds of formula I are prepared by reacting a compound of formula IV 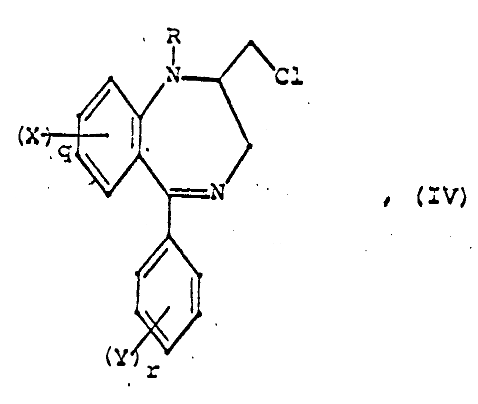
wherein
X, Y, q, r and R are as defined in formula I, with an amine of formula 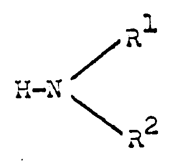
wherein
R¹ and R² are as defined in formula I, at temperatures of from 0°C to the boiling point of the amine in the presence of an acid-binding agent, or from 0°C to the boiling point of the solvent in the presence of an inert, aprotic solvent, yielding a compound of formula I.

After said processes the corresponding compounds of formulae Ia, Ib or I or acid addition salts thereof are isolated or optionally the compounds of formulae Ia or Ib or I are converted to the quaternary ammonium salts.

The process for the preparation of the compounds of formula I will be further illustrated by the following methods I and II which will be abbreviated in the following reaction scheme with MI and MII: 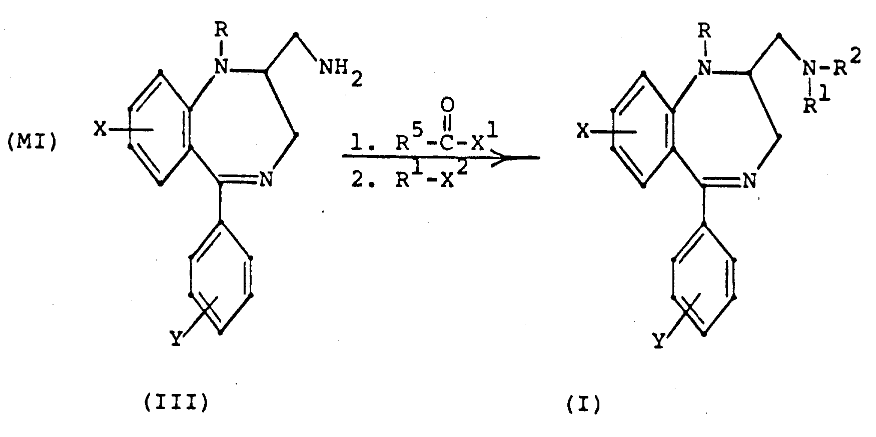
wherein X¹=OH, C1, 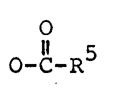
or 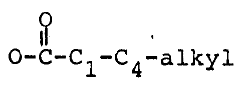
and X²=C1, Br, I, OSO₂C₁-C₄-alkyl, OSO₂-phenyl or OSO₂-substituted phenyl. 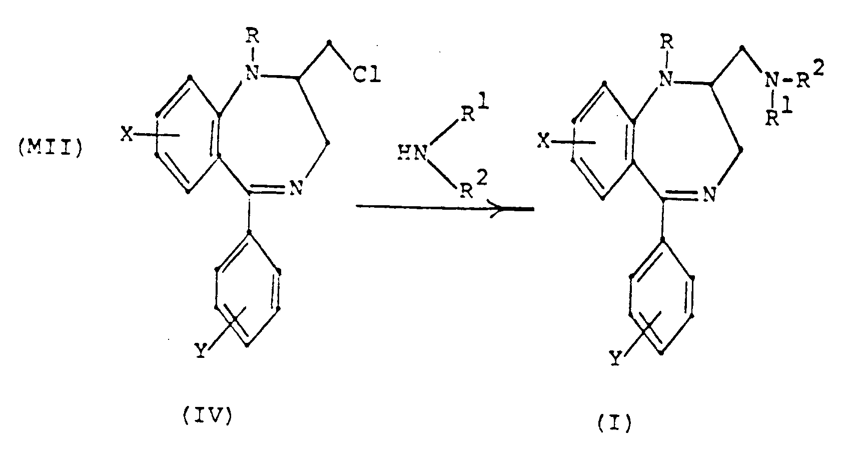
According to method MI, 2-substituted-aminomethyl-1,4-benzodiazepine derivatives of formula I may be prepared by acylating an amino compound of formula III, or an acid addition salt thereof, with a carboxylic acid or a reactive carboxylic acid derivative. (The preparation of 2-aminomethyl-1,4-benzodiazepine derivatives of formula III which are used as starting materials in the present invention nay be carried out in a known manner according to processes described in U.S. Patent 4,325,957 and German Offenlegungsschrift No. 2,221,558.) The acylaton is carried out in an aprotic solvent at temperatures between -30°C and the boiling point of the solvent under normal atmospheric pressure.

If a carboxylic acid halogenide or a carboxylic acid anhydride is used as the acylating agent, the reaction is preferably carried out in the presence of an acid-binding agent, such as a tertiary amine, for example, triethylamine, pyridine or 4-dimethylaminopyridine_{,} or an alkali metal hydroxide or alkali metal carbonate, for example, sodium hydroxide, potassium carbonate, and the like. Examples of suitable inert solvents include N,N-dimethylformamide, chloroform, methylene chloride, tetrahydrofuran, dioxane, toluene and chlorobenzene.

The compounds of formula I may also be prepared by reacting a compound of formula III with a carbonic acid, R⁵-CO₂H, in an inert solvent at temperatures of from -30°C to the boiling point of the solvent, preferably at room temperature, in the presence of a suitable coupling reagent, such as dicyclohexylcarbodiimide, 1-ethyl-3-(3-dimethylaminopropyl)carbodiimide hydrochloride or carbonyldiimidazole.

Compounds of formula I, wherein R¹ represents hydrogen, may subsequently be converted into the corresponding N-alkyl compounds by alkylation in the conventional manner. For example, such alkylations may be effected by reacting compounds of formula I, wherein R¹ is hydrogen, with a metallating agent, such as sodium or potassium hydride, or alkyl lithium reagent, in an inert solvent at temperatures of -78°C to the boiling point of the solvent. The metallated compound is then subsequently reacted with an alkyl halogenide, alkylsulfate or alkylsulfonic ester.

According to method MII, the compounds of formula I may alternatively be prepared by reacting a 2-halomethyl-1,4-benzodiazepine derivative of formula IV (prepared according to procedures in Eur. J. Med. Chem., 11, 501 (1976) and U.S. Patent 4,325,957) with an amine of the formula, HNR²R¹, in an inert solvent at temperatures of 0°C to the boiling point of the solvent, or without solvent, at temperatures of 0°C to the boiling point of the amine, HNR²R¹, in the presence of an acid-binding agent, such as an alkali metal hydroxide or alkali metal carbonate, as defined above.

A further object of the present invention is a process for preparing compounds of formula II 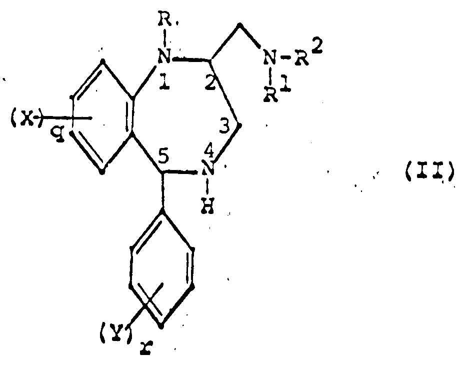
wherein
X, Y, q, r, R, R¹ and R² are as defined before.

According to said process a compound of formula I 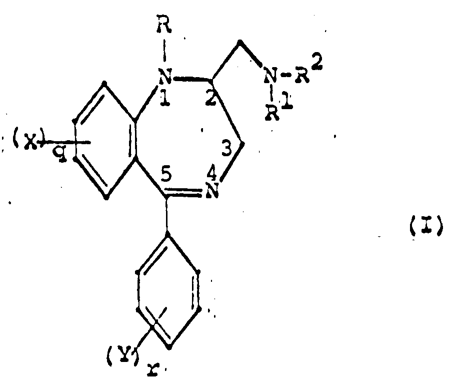
is dissolved in a suitable solvent, the solution is cooled to a temperature in the range of -30°C to + 15°C and the mixture treated with a reducing agent and stirred until the reduction reaction is completed and the resulting mixture poured into water and the compound of formula II extracted and washed. Thereafter the free amino compound of formula II or a salt thereof is isolated or the free amino compound of formula II is optionally converted into a quaternary ammonium salt.

The reaction accordingly is illustrated with the following method III which is abbreviated as MIII:

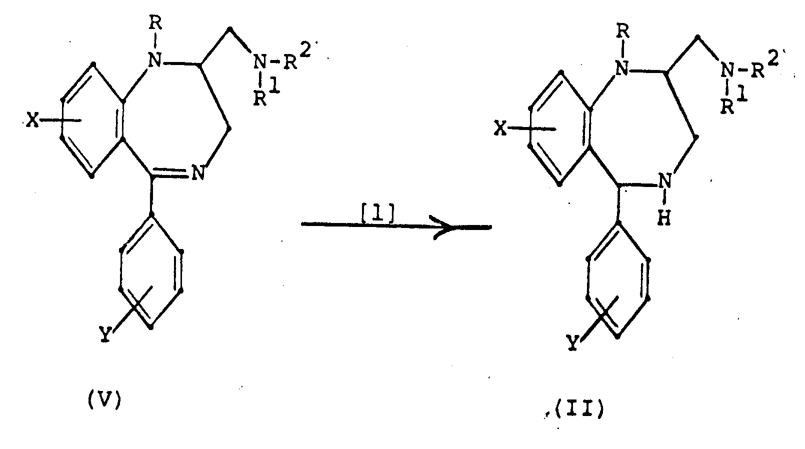

### [1] NaBH₃CN, NaBH₄ or LiBH₄

According to method MIII, 2-substituted-aminomethyl-1,4-benzodiazepine derivatives of formula II are prepared by dissolving a compound of formula V (prepared analogously to the preparation of compounds of formula III) in a suitable solvent, such as glacial acetic acid, methanol or ethanol, and cooling the solution is to -30° to + 15°C and treating it with a suitable reducing agent, such as sodium cyanoborohydride, sodium borohydride, or lithium borohydride. The reaction mixture is stirred until completion of the reaction (approximately 5 minutes to 5 hours) and poured into water. The resulting reaction mixture is extracted with an organic solvent, such as ethyl acetate, chloroform or methylene chloride, and the combined organic extracts are washed with sodium bicarbonate solution and brine. Concentration affords the crude product which may be further purified by chromatography or recrystallization.

The pharmaceutically-acceptable salts of the present invention may be synthesized from the compounds according to the instant invention which contain a basic or acidic moiety by conventional chemical methods. Generally, the salts are prepared by reacting the free base or free acid with stoichiometric amounts of or with an excess of the desired salt-forming inorganic or organic acid or base in a suitable solvent or in various combinations of solvents. The pharmaceutically-acceptable salts of the acids according to this invention are also readily prepared by conventional procedures such as treating an acid of this invention with an appropriate amount of a base, such as an alkali or alkaline earth metal hydroxide, e.g., sodium, potassium, lithium, calcium, or magnesium, or an organic base such as an amine, e.g., dibenzylethylenediamine, triethylamine, piperidine, pyrrolidine or benzylamine.

In addition to the racemic forms of the compounds of formulae I and II produced according to the foregoing preparation methods, the present invention also includes the optically-active forms of these compounds which may be obtained from the racemic mixtures in a conventional manner by formation of the salts using suitable optically-active acids, such as tartaric acid, O,O'-dibenzoyl tartaric acid, mandelic acid or di-O-isopropylidene-2-oxo-L-gulonic acid, and fractionated crystallization of the optically active antipodes of the resulting salts (see, S. W. Willen et al., Tetrahedron, 33, 2725-2736 (1977)). The salts may be transformed into free bases which may be further transformed into the pharmacologically-acceptable salts, and the racemic mixtures, optically-active isomers and acid-addition salts may be purified by recrystallization from solvents, such as lower alkyl alcohols or ethers. The preferred stereochemical configuration of the 2-position of all compounds according to the instant invention for CCK-antagonism is that designated (S) when R¹=H.

A further object of the present invention is a pharmaceutical composition which is an antagonist of the function of cholecystokinins in mammals and it is characterized in that it comprises as active ingredient a pharmaceutically effective amount of one or more 2-substituted-aminomethyl-1,4-benzodiazepine derivatives of formulae I or II, optical isomers of such derivatives, or pharmaceutically-acceptable salts or quaternary ammonium salts of said compounds and optionally a pharmaceutically acceptable carrier.

Preferably the corresponding pharmaceutical compositions further comprise an adjuvant.

The preferred active ingredients of said pharmaceutical compositions are the preferred compounds of formulae I and II defined before.

The inventive pharmaceutical compositions can be used to antagonize the function of cholecystokinins in the brain and in the gut. Therefore said compositions can be used for preventing or treating corresponding disorders of the gastrointestinal, central nervous and appetite regulatory systems of mammals, specially humans.

The screening of the inventive compounds of formulae I and II to determine their biological activity and obtain an IC₅₀ value for them, in order to identify significant CCK-antagonism, may be accomplished using an ¹²⁵-I-CCK receptor binding assay and in vitro isolated tissue preparations. These tests involve the following:

### CCK receptor binding (pancreas) method

CCK-33 was radiolabeled with ¹²⁵I-Bolton Hunter reagent (2000 Ci/mmole), as described by Sankara et al. (J. Biol. Chem., 254, 9349-9351, 1979). Receptor binding was performed according to Innis and Snyder (Proc. Natl. Acad. Sci., 77, 6917-6921, 1980), with the minor modification of adding the additional protease inhibitors, phenyl-methane sulfonyl fluoride and o-phenanthroline, which have no effect on the ¹²⁵I-CCK receptor binding assay.

The whole pancreas of a male Sprague-Dawley rat (200-350 g), which had been sacrificed by decapitation, was dissected free of fat tissue and homogenized in 20 volumes of ice-cold 50 mM Tris HCl (pH 7.7 at 25°C) with a Brinkmann Polytron PT-10. The homogenates were centrifuged at 48,000 g for 10 minutes, then the resulting pellets were resuspended in Tris Buffer, centrifuged as above, and resuspended in 200 volumes of binding assay buffer (50 mM Tris HCl, pH 7.7 at 25°C, 5 mM dithiothreitol, 0.1 mM bacitracin, 1.2 mM phenylmethane sulfonyl fluoride and 0.5 mN o-phenanthroline).

For the binding assay, 25 µl of buffer (for total binding), or unlabeled CCK-8 sulfate sufficient to give a final concentration of 1µM of CCK-8 (for nonspecific binding), or the compounds of the formula of the compounds according to the instant invention (for determination of antagonism to ¹²⁵I-CCK binding) and 25 µl of ¹²⁵I-CCK-33 (30,000-40,000 cpm), were added to 450 µl of the membrane suspensions in microfuge tubes. All assays were run in duplicate or triplicate, and the reaction mixtures were incubated at 37°C for 30 minutes and centrifuged in a Beckman Microfuge (4 minutes) immediately after adding 1 ml of ice-cold incubation buffer. The supernatant was aspirated and discarded, and the pellets were counted with a Beckman Gamma 5000. For Scatchard analysis to determine the mechanism of inhibition of ¹²⁵I-CCK binding by the most potent compounds (Ann. N.Y. Acad. Sci., 51, 660, 1949), ¹²⁵I-CCK-33 was progressively diluted with increasing concentrations of CCK-33.

### CCK receptor binding (brain) method

CCK-33 was radiolabeled and the binding was performed according to the description for the pancreas method, with modifications according to Saito et al., J. Neurochem., 37, 483-490, 1981.

Male Hartley guinea pigs (300-500 g) were sacrificed by decapitation, and the brains were removed and placed in ice-cold 50 mM Tris HCl (Trizma-7.4) [pH 7.4 at 25°C]. The cerebral cortex was dissected and used as a receptor source and each gram of fresh guinea pig brain tissue was homogenized in 10 ml of Tris/Trizma buffer with a Brinkmann polytron PT-10. The homogenates were centrifuged at 42,000g for 15 minutes, then the resulting pellets were resuspended in 80 volumes of binding assay buffer (10 mM N-2-hydroxy-ethyl-piperazine-N'-2-ethanesulfonic acid (HEPES), 5 mM MgCl₂, 1 mM ethylene glycol-bis- β-amino-ethyl-ether-N,N'-tetraacetic acid (EGTA), 0.4% BSA and 0.25 mg/ml bacitracin, pH 6.5).

The remainder of the binding assay method was as described for the pancreas method, except that the reaction mixtures were incubated at 25°C for 2 hours before centrifugation.

### In vitro effect of the compounds according to this invention on ¹²⁵I-CCK-33 receptor binding

The compounds of the instant invention inhibited specific ¹²⁵I-CCK-33 binding in a concentration-dependent manner, generally with an IC₅₀ less than or equal to 100 µM.

An additional method of confirming competitive antagonism of CCK which may be used is the following:

### Isolated guinea pig gall bladder method

The two halves of the gall bladders, free of adjacent tissue, of male Hartley guinea pigs (400-600g), which had been sacrificed by decapitation, are suspended under 1g tension along the axis of the bile duct in 5 ml organ bath, containing a Kreb's bicarbonate solution of 118 mM NaCl, 4.75 mM KCl, 2.54 CaCl₂, 1.19 mM KH₂PO₄, 1.2 mM MgSO₄, 25 mM NaHCO₃ and 11 mM dextrose, which is maintained at 32°C and bubbled with a mixture of 95% O₂ and 5% CO₂. The tissues are washed every 10 minutes for 1 hour to obtain equilibrium prior to the beginning of the study and the isometric contractions of the strips are recorded using Statham (60g:0.12 mm) strain gauges and a Hewlett-Packard 77588 recorder.

CCK-8 is added cumulatively to the baths and EC₅₀'s are determined using regression analysis. After washout (every 10 minutes for 1 hour), the compound to be tested is added at least 5 minutes before the addition of CCK-8 and the EC₅₀ of CCK-8 in the presence of compound to be tested is similarly determined.

A shift to the right of the CCK dose response curve without reduction of the maximal contractile response, indicates competitive antagonism of CCK from this method.

The ability of the compounds of the instant invention to antagonize CCK makes these compounds useful as pharmaceutical agents for mammals, especially for humans, for the treatment and prevention of disorders wherein CCK may be involved. Examples of such disease states include gastrointestinal disorders, especially such as irritable bowel syndrome or ulcers, excess pancreatic or gastric secretion, acute pancreatis, or motility disorders; central nervous system disorders, caused by CCK interactions with dopamine, such as neuroleptic disorders tardive dyskinesia, Parkinson's disease, psychosis or Gilles de la Tourette Syndrome; and disorders of appetite regulatory systems.

The compounds of the instant invention or pharmaceutically-acceptable salts thereof, may be administered to a human subject either alone or, preferably, in combination with pharmaceutically-acceptable carriers or diluents, in a pharmaceutical composition, according to standard pharmaceutical practice. The compounds can be administered orally or parenterally, including intravenous, intramuscular, intraperitoneal, subcutaneous and topical administration.

For oral use of an antagonist of CCK, according to this invention, the selected compounds may be administered, for example, in the form of tablets or capsules, or as an aqueous solution or suspension. In the case of tablets for oral use, carriers which are commonly used include lactose and corn starch, and lubricating agents, such as magnesium stearate also, are commonly added. For oral administration in capsule form, useful diluents include lactose and dried corn starch. When aqueous suspensions are required for oral use, the active ingredient is combined with emulsifying and suspending agents. If desired, certain sweetening and/or flavoring agents may be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled in order to render the preparation isotonic.

When a compound according to the instant invention, or a salt thereof, is used as an antagonist of CCK in a human subject, the daily dosage will normally be determined by the prescribing physician. Moreover, the dosage will vary according to the age, weight, and response of the individual patient, as well as the severity of the patient's symptoms. However, in most instances, an effective daily dosage will be in the range of from about 0.05 mg/kg to about 100 mg/kg, and preferably, of from 0.5 mg/kg to about 20 mg/kg, administered in single or divided doses. In some cases, however, it may be necessary to use dosages outside these limits.

The invention is further defined by reference to the following examples which is intended to be illustrative and not limiting.

### EXAMPLES 1

### Preparation of 1-methyl-2-(2'-indolecarbonyl)aminomethyl-5-(2'-fluorophenyl)-2,3-dihydro-1H-1,4-benzodiazepine.

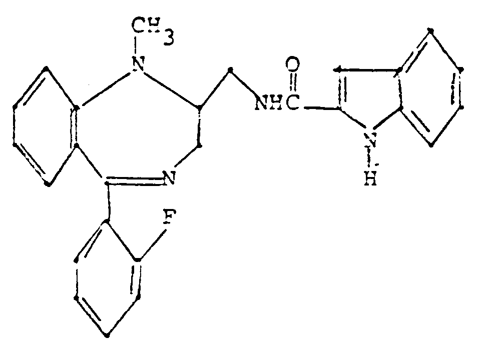

1-Methyl-2-aminomethyl-5-(2'-fluorophenyl)-2,3-dihydro-1H-1,4-benzodiazepine (250 mg, 0.88 mmole) and 2-indole carboxylic acid (142 mg, 0.88 mmole) were combined with 5 ml of dry methylene chloride, and 1-ethyl-3-(3-dimethylaminopropyl)carbodiimide hydrochloride (169 mg, 0.88 mmole) was added to this mixture at room temperature. The pH of the reaction mixture was adjusted to 8.5 with triethylamine and after overnight stirring, the reaction mixture was diluted with ethyl acetate (200 ml) and the organic phase was washed with saturated sodium bicarbonate solution and brine. Rotoevaporation of the dried (MgSO₄) extracts afforded 300 mg of an oil which was purified by silica gel chromatography (ethyl acetate-hexane elution, 2:1 v/v) to give the analytical sample (150 mg) which was 99% pure by HPLC.
MS (70 ev): 426 (M⁺), 253, 225, 144.
Pmr (CDCl₃): according to theory.

| Elemental Analysis: C₂₆H₂₃FN₄O 0.2H₂O: | | | |
|---|---|---|---|
| Calc: | N, 13.02; | C, 72.60; | H, 5.48 |
| Found: | N, 12.41; | C, 72.75; | H, 5.43. |

### EXAMPLE 2

### Preparation of 1-methyl-2-(4-thianaphthenemethylcarbonyl)aminomethyl-5-(2'-fluorophenyl)-2,3-dihydro-1H-1,4-benzodiazepine.

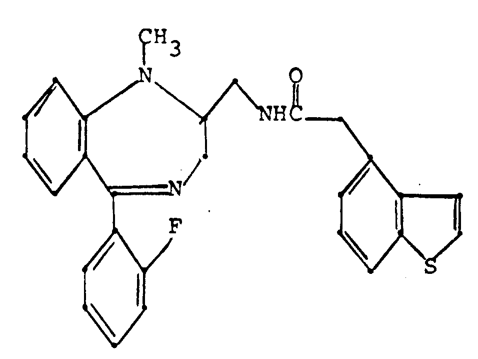

According to the method of Example 1 1-methyl-2-aminomethyl-5-(2'-fluorophenyl)-2,3-dihydro-1H-1,4-benzodiazepine (250 mg, 0.88 mmole) and 4-thianaphthene acetic acid (170 mg, 0.88 mmole) were combined with 4 ml of dry methylene chloride, and 1-ethyl-3-(3-dimethylaminopropyl)carbodiimide hydrochloride (169 mg, 0.88 mmole) was added to this mixture. after pH-adjustment, overnight stirring, dilution and washing, rotoevaporation of the dried extracts of the reaction afforded 300 mg of an oil which was purified by silica gel chromatography (ethyl acetate-hexane elution, 4:1 v/v) to give the analytical sample (100 mg) which was 88% pure by HPLC.
MS (FAB): 458 (M⁺+H), 253, 147.
Pmr (CDCl₃): according to theory.

| Elemental Analysis: C₂₇H₂₄FN₃OS 0.2H₂O: | | | |
|---|---|---|---|
| Calc: | N, 9.11; | C, 70.31; | H, 5.33 |
| Found: | N, 8.82; | C, 70.27; | H, 5.27. |

### EXAMPLE 3

### Preparation of 1-methyl-2-(2-L-hydroxy-2-phenylacetyl)aminomethyl-5-(2'-fluorophenyl)-2,3-dihydro-1H-1,4-benzodiazepine.

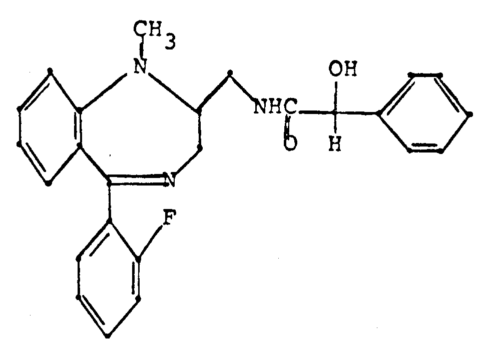

According to the method of Example 1, 1-methyl-2-aminomethyl-5-(2'-fluorophenyl)-2,3-dihydro-1H-1,4-benzodiazepine (500 mg, 1.76 mmole) and L-mandelic acid (268 mg, 1.76 mmole) were combined with 5 ml of dry methylene chloride, and 1-ethyl-3-(3-dimethylaminopropyl)carbodiimide hydrochloride (337 mg, 1.76 mmole) was added to this mixture. After pH-adjustment, overnight stirring, dilution (with 250 ml of ethyl acetate) end washing, rotoevaporation of the dried extracts of the reaction afforded 540 mg of an oil which was purified by silica gel chromatography (chloroform-ethanol-ammonia elution, 95:5:0.05 v/v) to give the analytical sample which was 94% pure by HPLC.
MS (20 ev): 417 (M⁺), 310, 253, 225.
Pmr (CDCl₃): according to theory.

| Elemental Analysis: C₂₅H₂₄FN₃O₂ 0.2H₂O: | | | |
|---|---|---|---|
| Calc: | N, 9.98; | C, 71.30; | H, 5.84 |
| Found: | N, 9.80; | C, 71.31; | H, 5.93. |

### EXAMPLE 4

### Preparation of 1-methyl-2-(1H-indol-3-yl)methylcarbonylaminomethyl-5-(2'-fluorophenyl)-2,3-dihydro-1H-1,4-benzodiazepine hydrate.

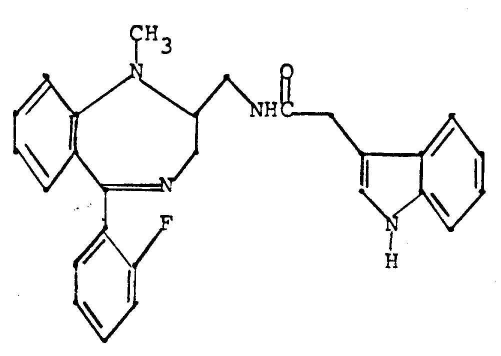

According to the method of Example 1, 1-methyl-2-aminomethyl-5-(2'-fluorophenyl)-2,3-dihydro-1H-1,4-benzodiazepine (250 mg, 0.88 mmole) and indole-3-acetic acid (154 mg, 0.88 mmole) were combined with 4 ml of dry methylene chloride, and 1-ethyl-3-(3-dimethylaminopropyl)carbodiimide hydrochloride (169 mg, 0.88 mmole) was added to this mixture. After pH-adjustment, overnight stirring, dilution and washing, rotoevaporation of the dried extracts of the reaction afforded 290 mg of an oil which was purified by silica gel chromatography (ethyl acetate elution) to give material which was 70% pure by HPLC. Rechromatography (chloroform-ethanol elution, 95:5 v/v) afforded the analytical sample, 93% pure, as a yellow solid.
MS (20 ev): 440 (M⁺), 253, 225, 130.
Pmr (CDCl₃): according to theory.

| Elemental Analysis: C₂₇H₂₅FN₄O H₂O: | | | |
|---|---|---|---|
| Calc: | N, 12.22; | C, 70.72; | H, 5.93 |
| Found: | N, 12.23; | C, 70.89; | H, 5.62. |

### EXAMPLE 5

### Preparation of 1-methyl-2-[1-(S)-1-methoxycarbonyl-2-phenylethylamino]methyl-5-(2'-fluorophenyl)-2,3-dihydro-1H-1,4-benzodiazepine

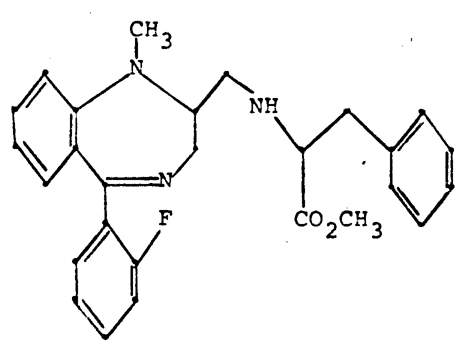

1-Methyl-2-chloromethyl-5-(2'-fluorophenyl)-2,3-dihydro-1H-1,4-benzodiazepine (150 mg, 0.5 mmole) and methyl-2(S)-amino-3-phenylpropionate (108 mg, 0.5 mmole) were combined in 4 ml of dry N,N-dimethylformamide, and potassium carbonate (138 mg, 1 mmole) and sodium iodide (70 mg, 0.5 mmole) were added to this mixture. The reaction mixture was protected from moisture and heated at 60°C for 48 hours. The solvent was then removed under reduced pressure and the residue was partitioned between ethyl acetate (100 ml) and saturated sodium bicarbonate solution (50 ml). The phases were separated and the organic layer was washed with sodium bicarbonate solution and brine, then dried (MgSO₄) and concentrated to yield 300 mg of crude product. The analytical product was obtained via chromatography on silica gel (ethyl acetate-hexane elution, 7:3 v/v) as a mixture of diasteriomers; 95% pure by HPLC.
MS (20 ev): 445 (M⁺), 253, 225, 212, 83.
Pmr (CDCl₃): according to theory.

| Elemental Analysis: C₂₇H₂₈FN₃O₂ 0.6H₂O: | | | |
|---|---|---|---|
| Calc: | N, 9.20; | C, 71.05; | H, 6.45 |
| Found: | N, 8.81; | C, 71.01; | H, 6.56. |

### EXAMPLE 6

### Preparation of 1-methyl-2-[2-((1,1-dimethylethoxy)carbonyl)amino-3-(1H-indol-3-yl)propanoyl]aminomethyl-5-(2'-fluorophenyl)-2,3-dihydro-1H-1,4-benzodiazepine.

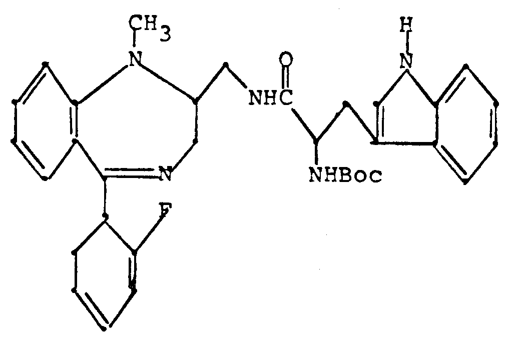

According to the method of Example 1, 1-methyl-2-aminomethyl-5-(2'-fluorophenyl)-2,3-dihydro-1H-1,4-benzodiazepine (250 mg, 0.88 mmole) and L-2-((1,1-dimethylethoxy)carbonyl)amino-3-(1H-indol-3-yl)propanoic acid (268 mg, 0.88 mmole) were combined with 4 ml of dry methylene chloride, and 1-ethyl-3-(3-dimethylaminopropyl)carbodiimide hydrochloride (169 mg, 0.88 mmole) was added to this mixture. After pH-adjustment, overnight stirring, dilution and washing, rotoevaporation of the dried extracts of the reaction afforded 500 mg of a foam which was purified by silica gel chromatography (chloroform-ethanol-ammonia elution, 90:10:1 v/v) to give the analytical sample (270 mg) which was 98% pure by HPLC; m.p. 124°C.
MS (FAB): 570 (M⁺+H), 514, 253.
Pmr (CDCl₃): according to theory.

| Elemental Analysis: C₃₃H₃₆FN₅O₃ 0.3H₂O: | | | |
|---|---|---|---|
| Calc: | N, 12.17; | C, 68.91; | H, 6.42 |
| Found: | N, 12.15; | C, 68.91; | H, 6.71. |

### EXAMPLE 7

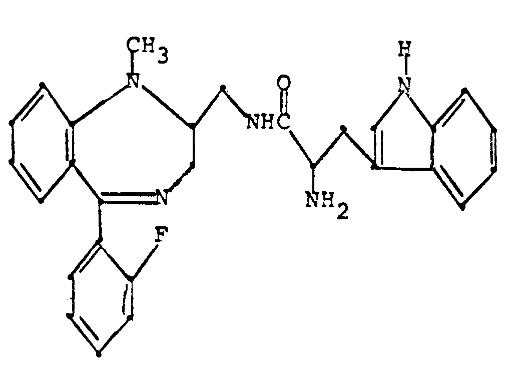

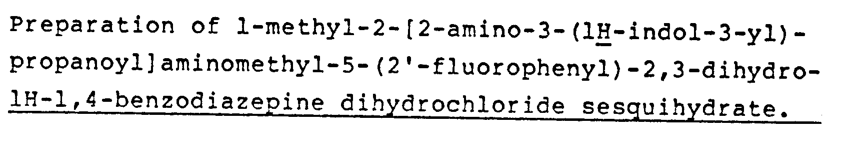

1-Methyl-2-[2-((1,1-dimethylethoxy)carbonyl)amino-3-(1H-indol-3-yl)propanoyl]aminomethyl-5-(2'-fluorophenyl)-2,3-dihydro-1H-1,4-benzodiazepine (50 mg, 0.08 mmole) was dissolved in 2 ml of ethyl acetate, cooled to 0°C and treated with hydrogen chloride gas for 1 hour. The solvent and excess hydrogen chloride were removed under reduced pressure to give the product as a foam which was 96% pure by HPLC.
MS (FAB): 470 (M⁺+H), 185.
Pmr (CD₃OD): according to theory.

| Elemental Analysis: C₂₈H₃₀Cl₂FN₅O 1.5H₂O: | | | |
|---|---|---|---|
| Calc: | N, 12.30; | C, 59.04; | H, 5.79 |
| Found: | N, 11.67; | C, 59.23; | H, 5.89. |

### EXAMPLE 8

### Preparation of 1-methyl-2-(2-methoxy-2-trifluoromethyl-2-phenylacetyl)aminomethyl-5-(2'-fluorophenyl)-2,3-dihydro-1H-1,4-benzodiazepine.

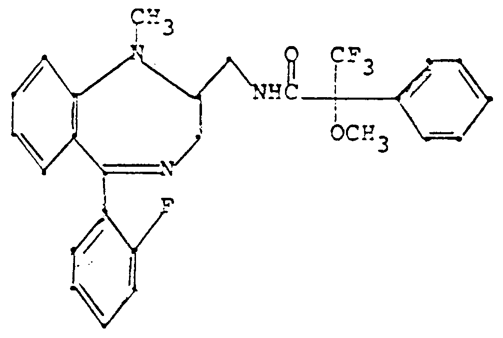

According to the method of Example 1, 1-methyl-2-aminomethyl-5-(2'-fluorophenyl)-2,3-dihydro-1H-1,4-benzodiazepine (250 mg, 0.88 mmole) and (-)-L-methoxy-L-(trifluoromethyl)phenylacetic acid (222 mg, 0.95 mmole) were combined with 4 ml of dry methylene chloride, and 1-ethyl-3-(3-dimethylaminopropyl)carbodiimide hydrochloride (182 mg, 0.95 mmole) was added to the mixture. After pH-adjustment, overnight stirring, dilution and washing, rotoevaporation of the dried extracts afforded 400 mg of a solid, as a mixture of diasteriomers, which was purified by silica gel chromatography (ethyl acetate-hexane elution, 2:3 v/v) to give the analytical sample (200 mg) which was pure by HPLC.
MS (20 ev): 499 (M⁺), 253, 225, 189.
Pmr (CDCl₃): according to theory.

| Elemental Analysis: C₂₇H₂₅F₄N₃O₂ 0.75H₂O: | | | |
|---|---|---|---|
| Calc: | N, 8.19; | C, 63.21; | H, 5.20 |
| Found: | N, 8.08; | C, 63.12; | H, 4.99. |

### EXAMPLE 9

### Preparation of 1-methyl-2-[2(S)-((1,1-dimethylethoxy)carbonyl)amino-3-acetamidomethylmercaptopropanoyl]aminomethyl-5-(2'-fluorophenyl)-2,3-dihydro-1H-1,4-benzodiazepine hemihydrate.

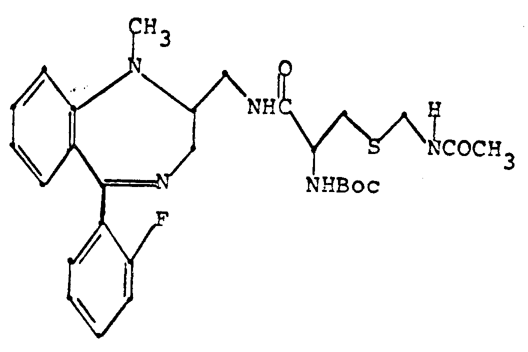

According to the method of Example 1, 1-methyl-2-aminomethyl-5-(2'-fluorophenyl)-2,3-dihydro-1H-1,4-benzodiazepine (620 mg, 2.18 mmole) and 2(S)-((1,1-dimethylethoxy)carbonyl)amino-3-acetamidomethylmercaptopropanoic acid (643 mg, 2.20 mmole) were combined with 10 ml of dry methylene chloride, and 1-ethyl-3-(3-dimethylaminopropyl)carbodiimide hydrochloride (422 mg, 2.20 mmole) was added to the mixture. After pH-adjustment, overnight stirring, dilution and washing, rotoevaporation of the dried extracts afforded 1 g of crude product which was purified by silica gel chromatography (chloroform-ethanol elution, 94:6 v/v) to give the analytical sample (420 mg) which was 96% pure by HPLC, m.p. 100-103°C.
MS (FAB): 558 (M⁺+H).
Pmr (CDCl₃): according to theory.

| Elemental Analysis: C₂₈H₃₆FN₅O₄S 0.5H₂O: | | | |
|---|---|---|---|
| Calc: | N, 12.36; | C, 59.34; | H, 6.58 |
| Found: | N, 12.47; | C, 59.14; | H, 6.66. |

### EXAMPLE 10

### Preparation of 1-methyl-2-benzoylsuccinoylaminomethyl-5-(2'-fluorophenyl)-2,3-dihydro-1H-1,4-benzodiazepine.

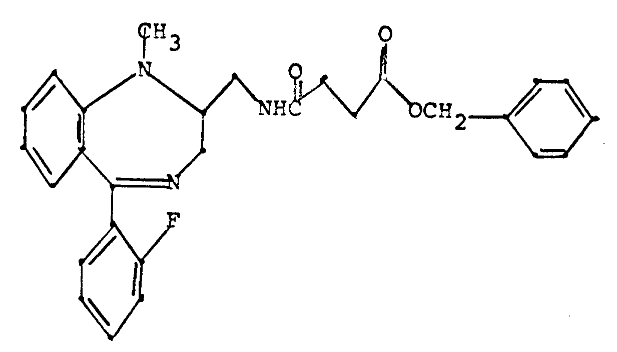

According to the method of Example 1, 1-methyl-2-aminomethyl-5-(2'-fluorophenyl)-2,3-dihydro-1H-1,4-benzodiazepine (250 mg, 0.88 mmole) and benzylsuccinic acid (185 mg, 0.88 mmole) were combined with 5 ml of dry methylene chloride, and 1-ethyl-3-(3-dimethylaminopropyl)carbodiimide hydrochloride (169 mg, 0.88 mmole) was added to the mixture. After pH-adjustment, overnight stirring, dilution and washing, rotoevaporation of the dried extracts afforded 360 mg of an oil which was purified by silica gel chromatography (chloroform-ethanol elution, 95:5 v/v) to give the analytical sample (140 mg) which was 88% pure by HPLC.
MS (20 ev): 473 (M⁺), 365, 253, 238, 225, 108.
Pmr (CDCl₃): according to theory.

| Elemental Analysis: C₂₈H₂₈FN₃O₃ 0.3H₂O: | | | |
|---|---|---|---|
| Calc: | N, 8.77; | C, 70.21; | H, 6.01 |
| Found: | N, 8.93; | C, 70.35; | H, 6.08. |

### EXAMPLE 11

### Preparation of 1-methyl-2-(acetamidomethylmercaptoacetyl)aminomethyl-5-(2'-fluorophenyl)-2,3-dihydro-1H-1,4-benzodiazepine hydrate.

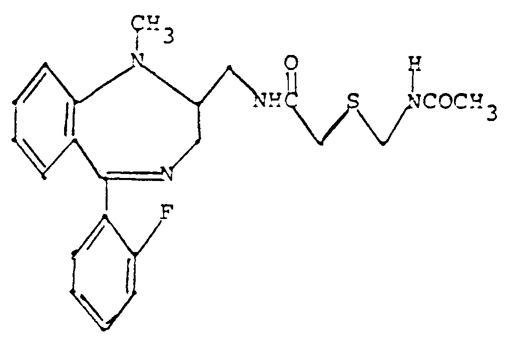

According to the method of Example 1, 1-methyl-2-aminomethyl-5-(2'-fluorophenyl)-2,3-dihydro-1H-1,4-benzodiazepine (1.96g, 6.9 mmole) and acetamidomethylmercaptoacetic acid (1.26 g, 7.7 mmole) were combined with 25 ml of dry methylene chloride and 1-ethyl-3-(3-dimethylaminopropyl)carbodiimide hydrochloride (1.48 g, 7.7 mmole) was added to the mixture. After pH-adjustment, overnight stirring, dilution with 250 ml of ethyl acetate and washing, rotoevaporation of the dried extracts afforded 2.63 g of an oil which was purified by silica gel chromatography (chloroform-ethanol-ammonia elution, 90:10:1 v/v) to give the analytical sample (850 mg) which was 95% pure by HPLC.
MS (30 ev): 428 (M⁺), 253, 225.
Pmr (CDCl₃): according to theory.

| Elemental Analysis: C₂₂H₂₅FN₄O₂S H₂O: | | | |
|---|---|---|---|
| Calc: | N, 12.54; | C, 59.17; | H, 6.09 |
| Found: | N, 12.68; | C, 59.37; | H, 5.89. |

In the following example 12 the compounds prepared according to the preceding examples 1 through 11 were tested as to their antagonism to CCK. Said tests showed that the inventive compounds are able to bind to CCK-receptors in the gut, i.e. in the pancreas, as well as in the brain:

### EXAMPLE 12

The tests were performed according to the CCK-receptor binding (pancreas) method described before and according to the CCK-receptor binding (brain) method described before. The corresponding tests are stated in the following table:

| Compound from Examples | IC₅₀ (µM) CCK, Pancreas | IC₅₀ (µM) CCK, Brain |
|---|---|---|
| 1 | 0.16 | 14.00 |
| 2 | 0.45 | 23.00 |
| 3 | 0.60 | ca.100 |
| 4 | 1.00 | 56.00 |
| 5 | 1.60 | ca.100 |
| 6 | 4.10 | 38.60 |
| 7 | 11.60 | ca.100 |
| 8 | 13.00 | ca.100 |
| 9 | 24.00 | ca.100 |
| 10 | 45.00 | 55.00 |
| 11 | 100.00 | ca.100 |

The IC₅₀ values (the concentrations required to cause inhibition of 50% of the ¹²⁵I-CCK binding) in the pancreas demonstrate the compounds of the present invention are from about 10 times to about 1000 times more potent than previously known compounds, and thus demonstrate significantly improved antagonism to the function of CCK.

## Claims

1. 2-Substituted aminomethyl-1,4-benzodiazepines having the formulae I , II or V 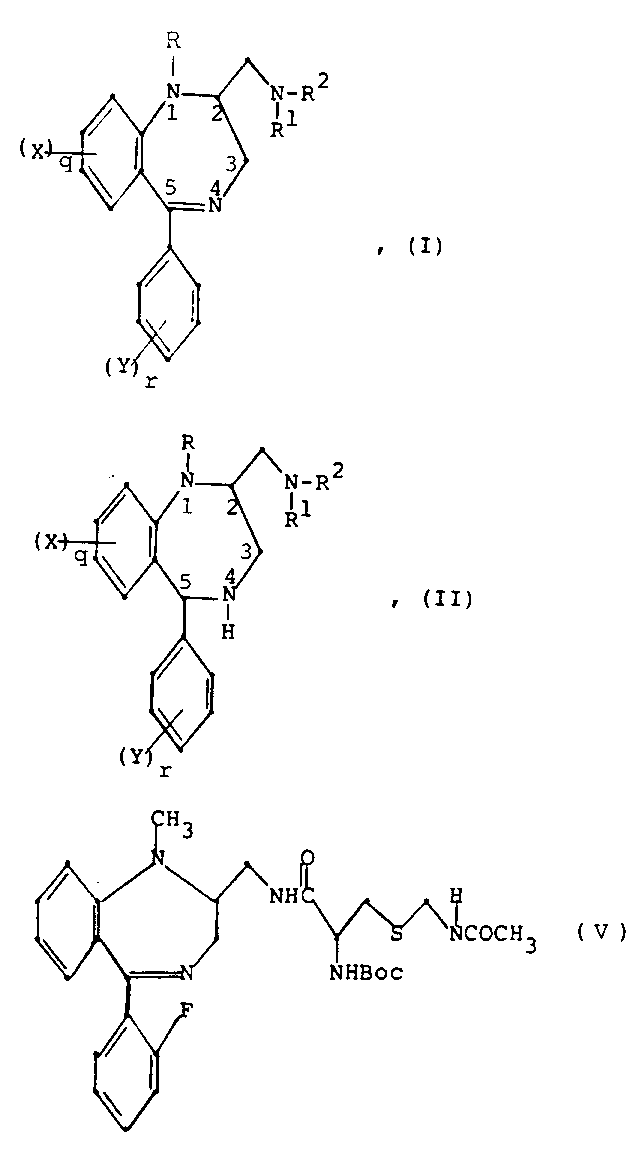 in which formulae
X is fluoro, chloro, bromo, alkyl having 1 - 4 carbon atoms, alkoxy having 1 - 4 carbon atoms, alkylthio having 1 - 4 carbon atoms, hydroxy, nitro, cyano, amino or trifluoro-methyl and the corresponding substituents X are attached at the position 7 and/or at the position 8;
q is zero, 1 or 2,
Y has the same meaning as X and the corresponding substituent may be attached at any of the positions 2 - 6 on the aromatic ring,
r is 1 or 2,
R is hydrogen, alkyl having 1 - 4 carbon atoms, alkenyl having up to 4 carbon atoms, cycloalkyl having 3 - 5 carbon atoms or acetyl,
R¹ is hydrogen, alkyl having 1 - 4 carbon atoms or cycloalkyl having 3 - 5 carbon atoms,
R² is either a substituted alkyl group of formula 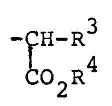 wherein
R³ is a group having the formulae
- (CH₂)ₙ-C₁-C₄-alkyl,
- (CH₂)ₙ-2-indole,
- (CH₂)ₙ-3-indole, or
- (CH₂)ₙ-phenyl
in which
n is 0 - 4 and
the phenyl is unsubstituted or mono- or disubstituted with substituents X which are as defined above and
R⁴ is hydrogen or alkyl having 1 - 4 carbon atoms or
the radical
R² is an acyl group having the formula 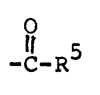 wherein the radical
R⁵ is one of the following groups:
a) 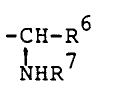
wherein
R⁶ is (CH₂)ₙ-2-indole or
(CH₂)ₙ-3-indole, in which group n is 0 - 4 and
R⁷ is hydrogen or a group of formulae COOR⁸, or 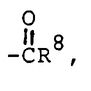 in which
R⁸ is an alkyl group having 1 - 4 carbon atoms,
b) -(CH₂)ₘSCH₂NHCOCH₃
wherein
m is 1 - 4
c) 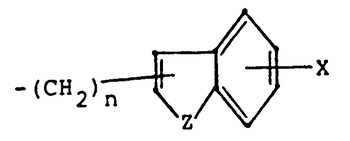
wherein
Z is O, S or NR,
wherein
R is as defined above, the group -(CH₂)ₙ- is attached at the 2- or 3-position,
n is 0 - 4 and
X is as defined above,
d) 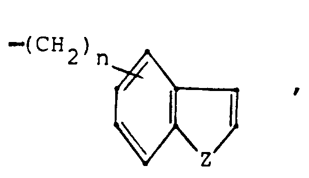
wherein
the group -(CH₂)ₙ- is attached at the 4- or 5-position and
Z and n are as defined in (c) above;
e) (CH₂)ₘCO₂CH₂phenyl,
wherein
m is 1 - 4,
f) -CHOHC₆H₅;
g) 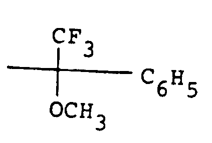 or
h) pyrazine, which is unsubstituted or mono-substituted, where the substituents may be Cl, COOR⁸, CN or NO₂ , wherein R⁸ is as defined in a) above;
and optical isomers of the compounds of formula I and salts or quaternary ammonium salts of the compounds of formulae I or II.

2. Compounds of formulae I or II according to claim 1 in which formulae
X is fluoro or chloro,
Y has the same meaning as X,
q and r are as defined in claim 1,
R is hydrogen or alkyl having 1 - 4 carbon atoms,
R¹ is hydrogen
R² is either a substituted alkyl group of formula 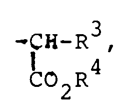 wherein
R³ is a group having the formulae
- (CH₂)ₙ-2-indole,
- (CH₂)ₙ-3-indole, or
- (CH₂)ₙ-phenyl
in which
n is 1 and the phenyl is unsubstituted or mono- or disubstituted with substituents X which are as defined above and
R⁴ is alkyl having 1 - 4 carbon atoms or the radical
R² is an acyl group having the formula 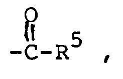 wherein the radical
R⁵ is one of the following groups:
a) 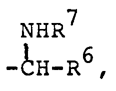
wherein
R⁶ is -CH₂-2-indole or
-CH₂-3-indole and
R⁷ is as defined in claim 1,
c) 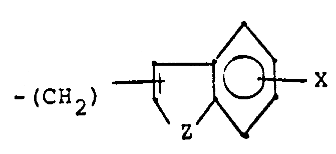
wherein Z and X are as defined in claim 1,
d) 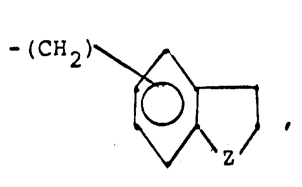
wherein
Z is as defined above in (c) and the group -CH₂- is attached at the 4- or 5-position or
f) -CHOHC₆H₅.

3. Compounds of formulae I or II according to claim 1 which are one of the following substances:
1-methyl-2-(2'-indolecarbonyl)aminomethyl-5-(2'-fluorophenyl)-2,3-dihydro-1H-1,4-benzodiazepine,
1-methyl-2-(4-thianaphthenemethylcarbonyl)aminomethyl-5-(2'-fluorophenyl)-2,3-dihydro-1H-1,4-benzodiazepine,
1-methyl-2-(2-L-hydroxy-2-phenylacetyl)aminomethyl-5-(2'-fluorophenyl)-2,3-dihydro-1H-1,4-benzodiazepine,
1-methyl-2-(1H-indol-3-yl)methylcarbonylaminomethyl-5-(2'-fluorophenyl)-2,3-dihydro-1H-1,4-benzodiazepine,
1-methyl-2-[1-(S)-1-methoxycarbonyl-2-phenylethylamino] methyl-5-(2'-fluorophenyl)-2,3-dihydro-1H-1,4-benzodiazepine,
1-methyl-2-[2-((1,1-dimethylethoxy)carbonyl)amino-3-(1H-indol-3-yl)propanoyl]aminomethyl-5-(2'-fluorophenyl)-2,3-dihydro-1H-1,4-benzodiazepine,
1-methyl-2-[2-amino-3-(1H-indol-3-yl)propanoyl]aminomethyl-5-(2'-fluorophenyl)-2,3-dihydro-1H-1,4-benzodiazepine,
1-methyl-2-(2-methoxy-2-trifluoromethyl-2-phenylacetyl)-aminomethyl-5-(2'-fluorophenyl)-2,3-dihydro-1H-1,4-benzodiazepine,
1-methyl-2-[2-(S)-[(1,1-dimethylethoxy)carbonyl)amino3-acetamidomethylmercapto-propanoyl]aminomethyl-5- (2'⁻fluorophenyl)-2,3-dihydro-1H-1,4-benzodiazepine,
1-methyl-2-benzylsuccinoyl-aminomethyl-5-(2'-fluorophenyl)-2,3-dihydro-1H-1,4-benzodiazepine,
1-methyl-2-(acetamidomethylmercapto-acetyl)aminomethyl-5-(2'-fluorophenyl)-2,3-dihydro-1H-1,4-benzodiazepine,
1-methyl-2-(4-thianaphthene-methylcarbonyl)aminomethyl-5-(2'-fluorophenyl)-2,3,4,5,-tetrahydro-1H-1,4-benzodiazepine,
1-methyl-2-(2-L-hydroxy-2-phenylacetyl)aminomethyl-5-(2'-fluorophenyl)-2,3,4,5-tetrahydro-1H-1,4-benzodiazepine or
1-methyl-2-(1H-indol-3-yl)methylcarbonylaminomethyl-5-(2'-fluorophenyl)-2,3,4,5-tetrahydro-1H-1,4-benzodiazepine.

4. A process for preparing 2-substituted-aminomethyl-1,4-benzodiazepines having the formula I: 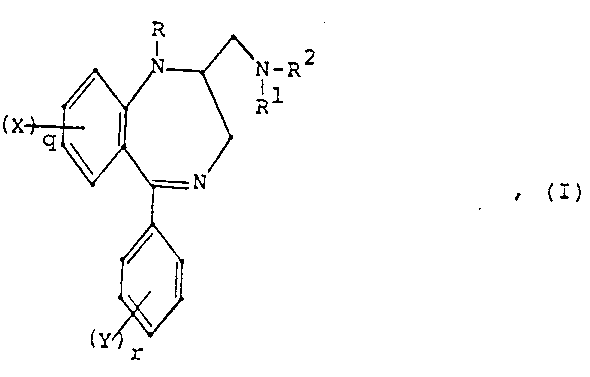 according to claim 1
wherein
X, Y, q, r, R, R¹ and R² are as defined in claim 1 wherein a compound of formula III 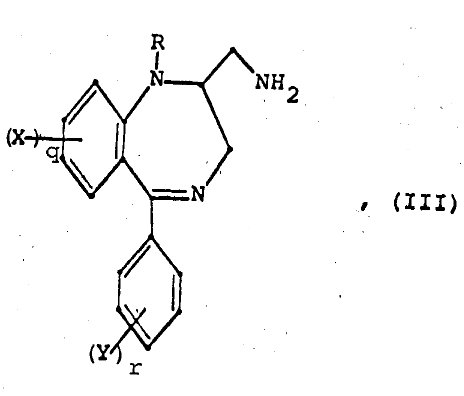 or an acid addition salt of the compound of formula III,
in which formula III X, Y, q, r and R are as defined in formula I,
is acylated with a carboxylic acid having the formula R⁵-COOH,
wherein
R⁵ is as defined in formula I,
or a derivative of said carboxylic acid, to yield compound of formula Ia 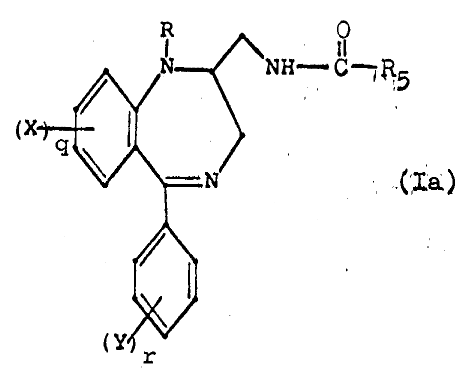 which compounds of formula Ia are optionally alkylated by substituting the hydrogen atom bonded to the nitrogen atom of the acid amide group by an alkyl group having 1 - 4 carbon atoms or a cycloalkyl group having 3 - 5 carbon atoms,
or
a compound of formula IV 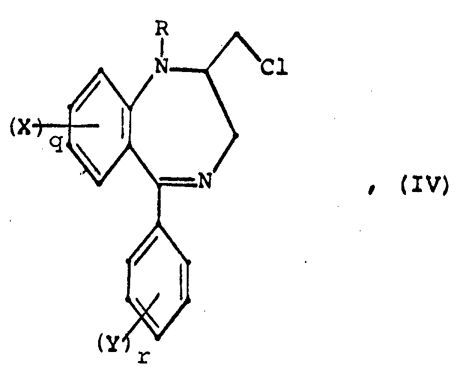 wherein
X, Y, q, r and R are as defined in formula I, is reacted with an amine of formula 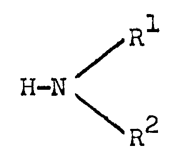 wherein
R¹ and R² are as defined in formula I,
at temperatures of from 0°C to the boiling point of the amine in the presence of an acid-binding agent, or from 0°C to the boiling point of the solvent in the presence of an inert, aprotic solvent, yielding a compound of formula I and that the amino compounds of formulae Ia or I or acid addition salts thereof are isolated or that optionally the compounds of formulae Ia or I are converted into quaternary ammonium salts.

5. Process according to claim 4. characterized in that the acylation reaction is carried out in an aprotic solvent which is selected from the group consisting of N,N-dimethylformamide, chloroform, methylene chloride, tetrahydrofuran, dioxane, toluene and chlorobenzene, at a temperature of from -30°C to the boiling point of the solvent, optionally in the presence of a suitable coupling reagent, which is selected from the group consisting of dicyclohexylcarbodiimide, 1-ethyl-3-(3-dimethylaminopropyl)carbodiimidehydrochloride and carbonyldiimidazole.

6. Process according to claim 5, wherein the acylation employs a carboxylic acid halogenide or carboxylic acid anhydride and the acylation is additionally carried out in the presence of an acid-binding agent.

7. Process according to claim 6, wherein the acid binding agent is selected from the group consisting of a tertiary amine, an alkali metal, hydroxide and an alkali metal carbonate.

8. Process according to claim 7, wherein the acid-binding agent is selected from the group consisting of triethylamine, pyridine, 4-dimethylaminopyridine, sodium hydroxide and potassium carbonate

9. Process for preparing compounds of formula II 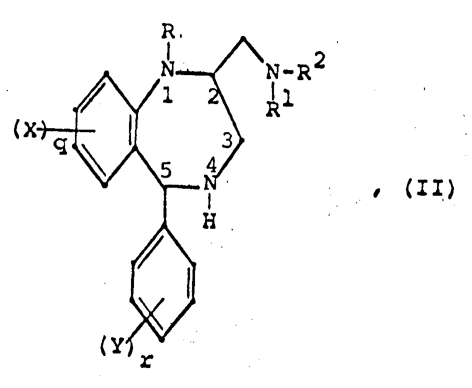 according to claim 1,
wherein
X, Y, q, r, R, R¹ and R² are as defined in claim 1, wherein a compound of formula I 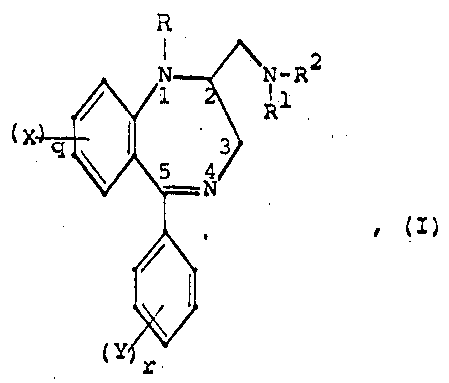 is dissolved in a suitable solvent, the solution is cooled to a temperature in the range of -30° C to + 15°C and the mixture treated with a reducing agent and stirred until the reduction reaction is completed and the resulting mixture poured into water and the compound of formula II extracted and washed and wherein the free amino compound of formula II or a salt thereof is isolated or the free amino compound of formula II is optionally converted into a quaternary ammonium salt.

10. Process according to claim 9 wherein the solvent used in the reduction reaction is glacial acetic acid, methanol or ethanol, and the reducing agent is sodium cyanoborohydride, sodium borohydride, or lithium borohydride.

11. Pharmaceutical composition which is an antagonist of the function of cholecystokinins in mammals characterized in that it comprises as active ingredient a pharmaceutically effective amount of one or more 2-substituted-aminomethyl-1,4-benzodiazepine derivatives according to claim 1, optical isomers of such derivatives, or pharmaceutically-acceptable salts or quaternary ammonium salts of said compounds and optionally a pharmaceutically acceptable carrier.

12. Pharmaceutical composition according to claim 11, further comprising an adjuvant.

13. Pharmaceutical composition according to claims 11 or 12 which comprises as active ingredient a compound according to claim 3 or a pharmaceutically acceptable salt of said compound.

14. Pharmaceutical composition according to one of the claims 11 - 13 characterized in that it contains the compounds according to claim 1 in such an amount that the composition can be used for the administration of 0,05 mg of said compound per kg. bodyweight to 100 mg of said substance per kg. bodyweight in a single dose or in divided doses.

15. Pharmaceutical composition according to claim 14 characterized in that it contains the pharmaceutically effective compound in such an amount that said compound can be administered in an amount of from 0,5 mg/kg bodyweight to 20 mg/kg bodyweight.

16. Pharmaceutical composition according to one of the claims 11 - 15 characterized in that it is a pharmaceutical composition for treating human beings.

## Patentansprüche

1. 2-substituierte Aminomethyl-1,4-benzodiazepine mit den Formeln I, II oder V 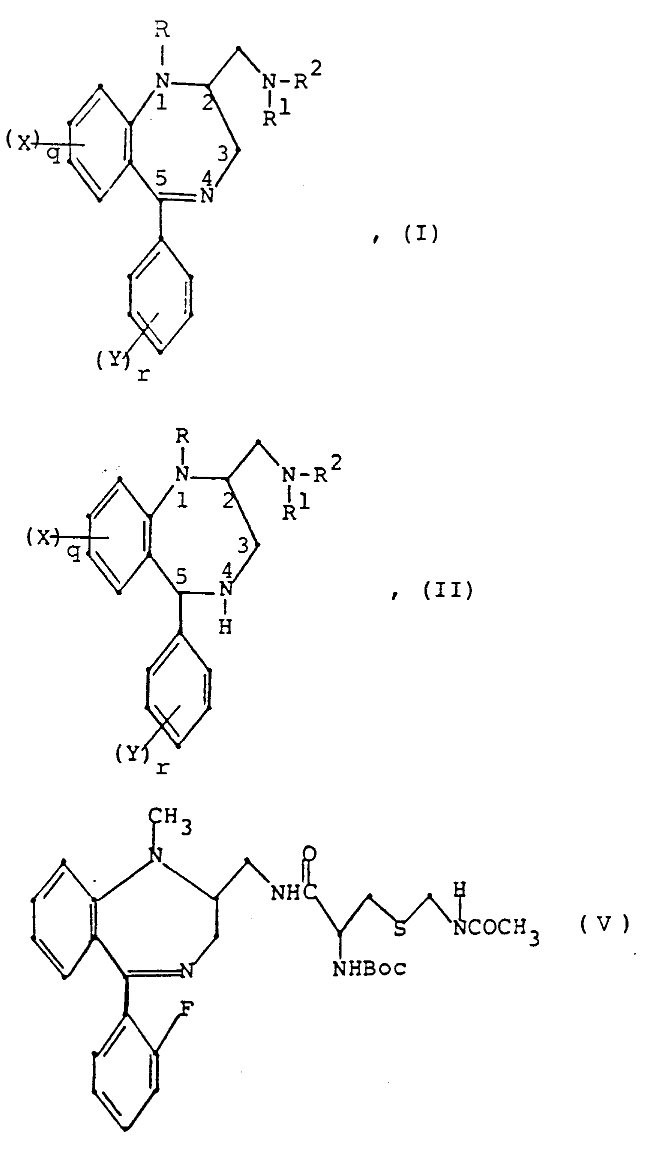 wobei in den Formeln
X Fluor, Chlor, Brom, Alkyl mit 1-4 Kohlenstoffatomen, Alkoxy mit 1-4 Kohlenstoffatomen, Alkylthio mit 1-4 Kohlenstoffatomen, Hydroxy, Nitro, Cyano, Amino oder Trifluormethyl bedeutet und die entsprechenden Substituenten X in 7- und/oder in 8-Stellung befestigt sind;
q Null, 1 oder 2 ist,
y dieselbe Bedeutung wie X hat und der entsprechende Substituent in einer der Stellungen von 2-6 am aromatischen Ring befestigt sein kann
r 1 oder 2 ist
R Wasserstoff, Alkyl mit 1-4 Kohlenstoffatomen, Alkenyl mit bis zu 4 Kohlenstoffatomen, Cycloalkyl mit 3-5 Kohlenstoffatomen oder Acetyl ist,
R¹ Wasserstoff, Alkyl mit 1-4 Kohlenstoffatomen oder Cycloalkyl mit 3-5 Kohlenstoffatomen ist,
R² entweder eine substituierte Alkylgruppe der Formel 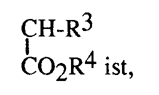 worin
R³ eine Gruppe mit den Formeln
- (CH₂)ₙ-C₁-C₄-alkyl,
- (CH₂)ₙ-2-indol,
- (CH₂)ₙ-3-indol oder
- (CH₂)ₙ-phenyl,
in denen n 0-4 ist und
das Phenyl mit den Substituenten X, die die oben gegebene Definition besitzen, unsubstituiert oder monosubstituiert oder disubstituiert ist und
R⁴ Wasserstoff oder Alkyl mit 1-4 Kohlenstoffatomen ist oder der Rest
R² eine Acylgruppe der Formel 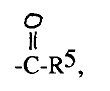 worin die Gruppe
R⁵ eine der folgenden Gruppen ist:
a) 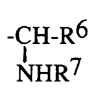
worin
R⁶ (CH₂)ₙ-2-indol oder
(CH₂)ₙ-3-indol ist, wobei in der Gruppe n 0-4 bedeutet und
R⁷ Wasserstoff oder eine Gruppe der Formeln
COOR⁸ oder 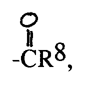 in denen
R⁸ eine Alkygruppe mit 1-4 Kohlenstoffatomen ist, ist,
b) -(CH₂)ₘSCH₂NHCOCH₃
worin
m 1-4 ist
c) 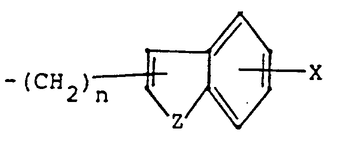
worin
Z O, S oder NR,
worin
R die oben gegebene Definition besitzt,
ist,
die Gruppe -(CH₂)ₙ- in 2- oder 3-Stellung befestigt ist,
n 0-4 ist und
X die oben gegebene Definition besitzt,
d) 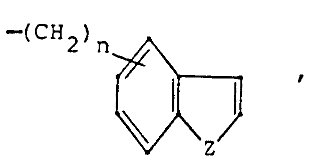
worin
die Gruppe -(CH₂)ₙ- in 4- oder 5-Stellung befestigt ist und
Z und n die in (c) oben gegebene Definition besitzen;
e) (CH₂)ₘCO₂CH₂phenyl
worin m 1-4 ist
f) -(CHOH)C₆H₅;
g) 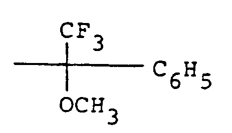
h) Pyrazin, das unsubstituiert oder monosubstituiert ist, worin die Substituenten Cl, COOR⁸, CN oder NO₂, worin R⁸ die in a) gegebene Definition besitzt, sein können;
und optische Isomere der Verbindungen der Formel I und Salze oder quaternäre Ammoniumsalze der Verbindungen der Formeln I oder II, sein können.

2. Verbindungen der Formeln I oder II nach Anspruch 1, wobei in den Formeln
X Fluor oder Chlor ist,
Y dieselbe Bedeutung wie X hat
q und r die in Anspruch 1 oben gegebene Definition besitzen,
R Wasserstoff oder Alkyl mit 1-4 Kohlenstoffatomen ist,
R¹ Wasserstoff ist,
R² entweder eine substituierte Alkylgruppe der Formel 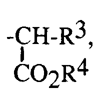 worin
R³ eine Gruppe mit den Formeln
- (CH₂)ₙ-2-indol,
- (CH₂)ₙ-3-indol oder
- (CH₂)ₙ-phenyl,
in denen
n 1 ist und
das Phenyl unsubstituiert oder mit den Substituenten X, die die oben gegebene Definition besitzen, mono- oder disubstituiert ist und
R⁴ Alkyl mit 1-4 Kohlenstoffatomen ist, oder
der Rest
eine Acylgruppe der Formel 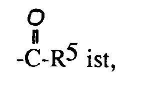 worin der Rest
R⁵ eine der folgenden Gruppen ist:
a) 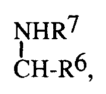
worin
R⁶ ein (CH₂)ₙ-2-indol oder ein (CH₂)ₙ-3-indol ist und
R⁷ die in Anspruch 1 gegebene Definition besitzt,
c) 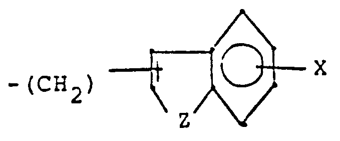 worin Z und X die in Anspruch 1 gegebene Definition besitzen
d) 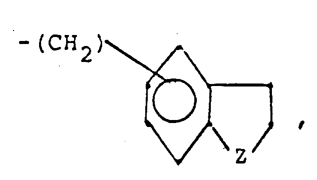
worin
Z die in (c) gegebene Definition besitzt und die Gruppe -CH₂- in der 4- oder 5-Stellung befestigt ist oder
f) -CHOHC₆H₅ ist, ist.

3. Verbindungen der Formeln I oder II nach Anspruch 1, die eine der fogenden Substanzen sind:
1-Methyl-2-(2'-indolcarbonyl)-aminomethyl-5-(2-'fluorphenyl)-2,3-dihydro-1H-1,4-benzodiazepin,
1-Methyl-2-(4-thianaphthenmethylcarbonyl)-aminomethyl-5-(2'-fluorphenyl)-2,3-dihydro-1H-1,4-benzodiazepin,
1-Methyl-2-(2-L-hydroxy-2-phenylacetyl)-aminomethyl-5-(2'-fluorphenyl)-2, 3-dihydro-1H-1,4-benzodiazepin,
1-Methyl-2-(1H-indol-3-yl)-methylcarbonylaminomethyl-5-(2'-fluorphenyl)-2,3-dihydro-1H-1,4-benzodiazepin,
1-Methyl-2-[1-(S)-1-methoxycarbonyl-2-phenylethylamino]-methyl-5-(2'-fluorphenyl)-2,3-dihydro-1H-1,4-benzodiazepin,
1-Methyl-2-[2-((1,1-dimethylethoxy)carbonyl)-amino-3-(1H-indol-3-yl)-propanoyl]aminomethyl-5-(2'-fluorphenyl)-2,3-dihydro-1H-1,4-benzodiazepin,
1-Methyl-2-[2-amino-3-(1H-indol-3-yl)-propanoyl]-aminomethyl-5-(2'-fluorphenyl)-2,3-dihydro-1H-1,4-benzodiazepin,
1-Methyl-2-[2-methoxy-2-trifluormethyl-2-phenylacetyl-)-aminomethyl-5-(2'-fluorphenyl)-2,3-dihydro-1H-1,4-benzodiazepin,
1-Methyl-2-[2-(S)-((1,1-dimethylethoxy)-carbonyl)-amino-3-acetamidomethylmercaptpropanoyl]aminomethyl-β-(2'-fluorphenyl)-2,3-dihydro-1H-1,4-benzodiazepin,
1-Methyl-2-[benzylsuccinoylaminomethyl-5-(2'-fluorphenyl-2,3-dihydro-1H-1,4-benzodiazepin,
1-Methyl-2-(acetamidoaminomethylmercaptoacetyl)-amionmethyl-5-(2-fluorphenyl-2,3-dihydro-1H-1,4-benzodiazepin,
1-Methyl-2-(4-thianaphthenmethylcarbonyl)-amionmethyl-5-(2-'-fluorphenyl)-2,3,4,5-tetrahydrohydro-1H-1,4-benzodiazepin,
1-Methyl-2-(2-L-hydroxy-2-phenylacetyl)-aminomethyl-5-(2-'-fluorphenyl)-2,3,4,5-tetrahydrohydro-1H-1,4-benzodiazepin oder
1-Methyl-2-(1H-indol-3-yl)-methylcarbonylaminomethyl-5-(2-'-fluorphenyl)-2,3,4,5-tetrahydrohydro-1H-1,4-benzodiazepin.

4. Verfahren zur Herstellung 2-substituierter Aminomethyl-1,4-benzodiazepine mit der Formel 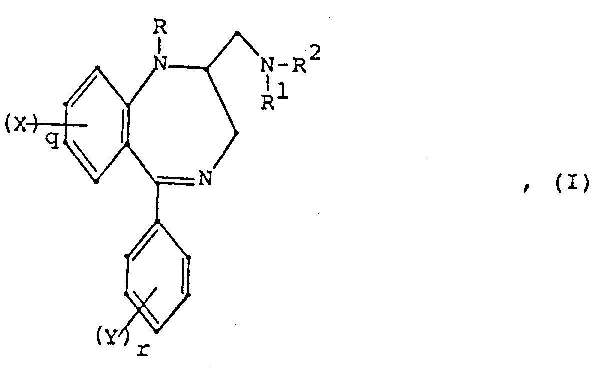 nach Anspruch 1
worin
x, Y, q,r, R, R¹ und R² die in Anspruch 1 gegebene Definition besitzten, worin eine Verbindung der Formel III 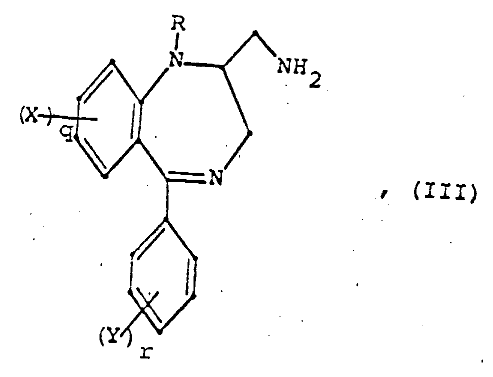 oder ein saures Additionssalz der Verbindung der Formel III, wobei in der Formel III X, Y, q, r und R die in Formel I gegebene Definition besitzten,
mit einem Carbonsäuresalz der Formel R⁵-COOH,
worin
R⁵ die in Formel I gegebene Definition besitzt,
oder ein Derivat der genannten Carbonsäure ist,acyliert ist um eine Verbindung der Formel Ia 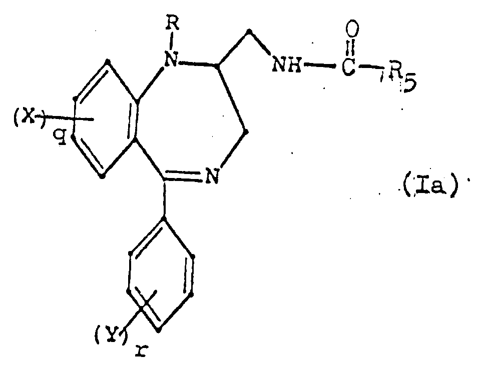 zu ergeben,
wobei die Verbindungen der Formel Ia gegebenfalls durch Substitution des an das Stickstoffatom der Säureamidgruppe gebundene Wasserstoffatoms durch eine Alkylgruppe mit 1-4 Kohlenstoffatomen oder eine Cycloalkylgruppe mit 3-5 Kohlenstoffatomen,alkyliert werden,
oder
eine Verbindung der Formel IV 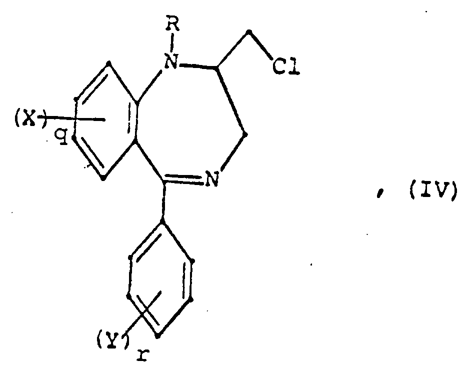 worin
X, Y, q, r und R die bei Formel I gegebene Definition besitzen, mit einem Amin der Formel 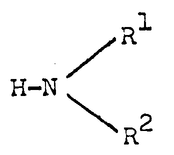 worin
R¹ und R² die bei Formel I gegebene Definition besitzen,
bei Temperaturen von 0° C bis zum Siedepunkt des Amins in Gegenwart eines Säurebindenden Mittels, oder von 0° C bis zum Siedepunkt des Lösungsmittels in Gegenwart eines inerten, aprotischen Lösungsmittels umgesetzt wird, wobei eine Verbindung der Formel I erhalten wird, und so, daß die Amino-Verbindungen der Forineln Ia oder I oder die sauren Additionssalze davon isoliert werden, oder daß gegebenfalls die Verbindungen der Formeln Ia oder I in quaternäre Amlnoniumsalze übergeführt werden.

5. Verfahren nach Anspruch 4, dadurch gekennzeichnet, daß die Acylierungsreaktion in einem aprotischen Lösungsmittel, das aus der Gruppe bestehend aus N,N-Dimethylformamid, Chloroform, Methylenchlorid, Tetrahydrofuran, Dioxan, Toluol und Chlorbenzol, bei einer Temperatur von -30° C bis zum Siedepunkt des Lösungsmittels, gegebenfalls in Gegenwart eines geeigneten Kupplungsreagens, das aus der Gruppe bestehend aus Dicyclohexylcarbodiimid, 1-Ethyl-3-(3-dimethylaminopropyl)carbodiimidhydrochlorid und Carbodiimidazol ausgewählt wird, durchgeführt wird.

6. Verfahren nach Anspruch 5, bei dein bei der Acylierung ein Carbonsäurehalogenid oder Carbonsäureanhydrid eingesetzt wird und die Acylierung zusätzlich in Gegenwart eines säurebindenden Mittels durchgeführt wird.

7. Verfahren nach Anspruch 6, bei dein das säurebindende Mittel aus der Gruppe bestehend aus einem tertiären Amin, einem Alkalimetallhydroxid und einem AIkalimetallcarbonat ausgewählt wird.

8. Verfahren nach Anspruch 7, bei dem das säurebindende Mittel aus der Gruppe bestehend aus Triethylamin, Pyridin, 4-Dimethylaminopyridin, Natriumhydroxid und Kaliumcarbonat ausgewählt wird.

9. Verfahren zur Herstellung von Verbindungen der Formel II 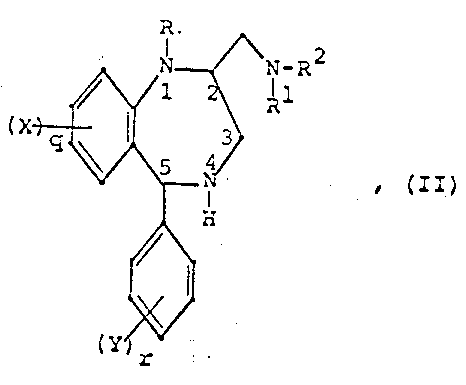 nach Anspruch 1,
worin
X, Y, q, r, R, R¹ und R² die in Anspruch 1 gegebene Definition besitzen, wobei eine Verbindung der Formel I 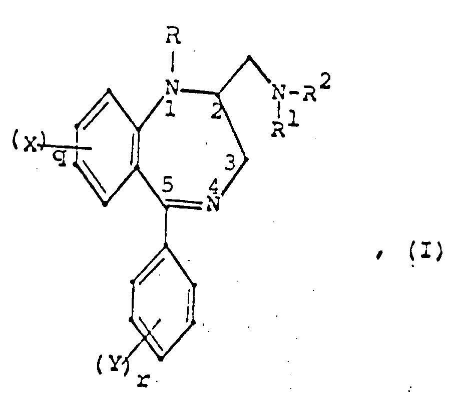 in einem geeigneten Lösungsmittel aufgelöst wird, die Lösung auf eine Temperatur im Bereich von -30° C bis + 15° C abgekühlt wird und das Gemisch mit einem reduzierenden Mittel behandelt wird, bis die Reduktionsreaktion vollendet ist und das erhaltene Gemisch in Wasser gegossen wird und die Verbindung der Formel II extrahiert und gewaschen wird und wobei die freie Amino-Verbindung der Formel II oder ein Salz davon isoliert wird oder die freie Aminosäure gegenbenfalls in ein quaternäres Ammoniumsalz übergeführt wird.

10. Verfahren nach Anspruch 9, bei dem das bei der Reduktionsreaktion verwendete Lösungsmittel Eisessig, Methanol oder Ethanol ist und das Reduktionsmittel Natriumcyanoborhydrid, Natriumborhydrid oder Lithiumborhydrid ist.

11. Pharmazeutisches Präparat, das ein Antagonist der Funktion der Cholecystokinine in Säugern ist, dadurch charakterisiert, daß es als aktiven Inhaltsstoff eine pharmazeutisch wirksame Menge eines oder mehrerer 2-substituierter Aminomethyl-1,4-benzodiazepin-Derivate nach Anspruch 1, optische Isomere solcher Derivate oder pharmazeutisch annehmbare Salze oder quaternäre Ammoniumsalze der genannten Verbindungen und gegebenfalls einen pharmazeutisch annehmbaren Trägerstoff enthält.

12. Pharmazeutisches Präparat nach Anspruch 11, das außerdem ein Adjuvans enthält.

13. Pharmazeutisches Präparat nach einem der Ansprüche 11 oder 12, das als aktiven Inhaltsstoff eine Verbindung nach Anspruch 3 oder ein pharmazeutisch annehmbares Salz der genannten Verbindung enthält.

14. Pharmazeutisches Präparat nach einem der Ansprüche 11-13, dadurch charakterisiert, daß es die Verbindungen nach Anspruch 1 in einer solchen Menge enthält, daß das Präparat zur Verabreichung von 0,05 mg der genannten Verbindung pro kg Körpergewicht bis 100 mg der genannten Substanz pro kg Körpergewicht in einer Einzeldosis oder in aufgeteileten Dosen verwendet werden kann.

15. Pharmazeutisches Präparat nach Anspruch 14, dadurch charakterisiert, daß es die pharmazeutisch wirksame Verbindung in einer solchen Menge enthält, daß die genannte Verbindung in einer Menge von 0,5 mg/kg Körpergewicht bis 20 mg/kg Körpergewicht verabreicht werden kann.

16. Pharmazeutisches Präparat nach einem der Ansprüche 11-15, dadurch charakterisiert, daß es ein pharmazeutisches Präparat zur Behandlung von Menschen ist.

## Revendications

1. Aminométhyl-1-4-benzodiazépines substituées en position 2, de formule I, II ou V 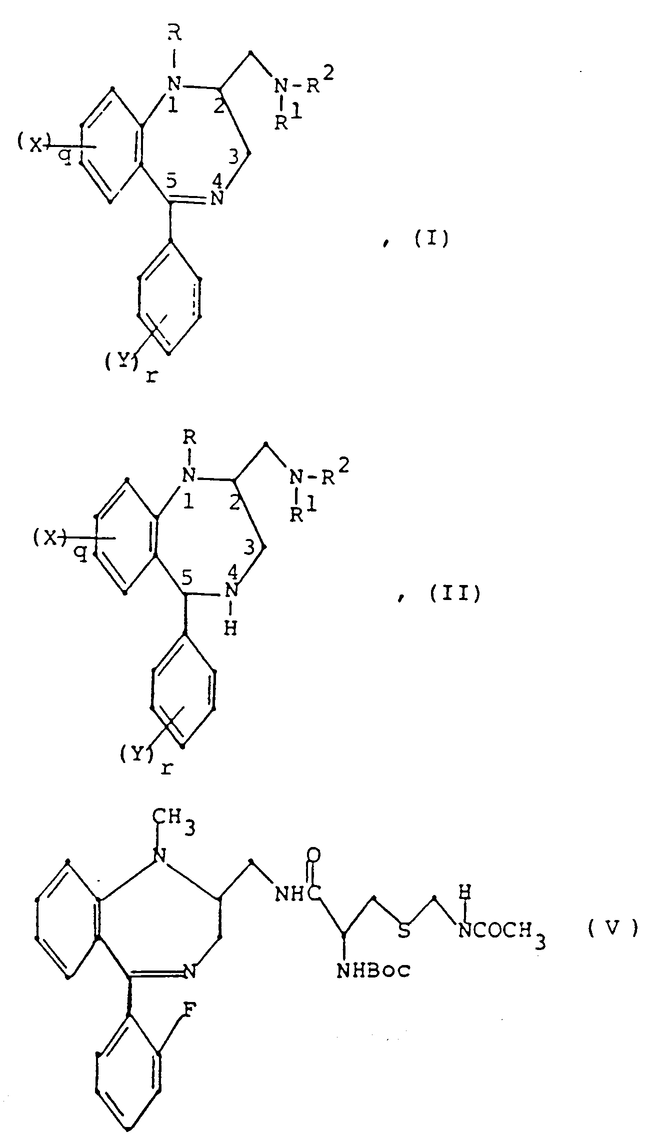 formules dans lesquelles
X est un groupe fluoro, chloro, bromo, alkyle comportant 1 - 4 atomes de carbone, alcoxy comportant 1 - 4 atomes de carbone, alkylthio comportant 1 - 4 atomes de carbone, hydroxy, nitro, cyano, amino ou trifluorométhyle et les substituants X correspondants sont fixés sur la position 7 et/ou sur la position 8;
q est égal à zéro, 1 ou 2,
Y a la même signification que X et le substituant correspondant peut être fixé sur l'une quelconque des positions 2 - 6 du noyau aromatique,
r est égal à 1 ou 2,
R est un hydrogène, un groupe alkyle comportant 1 - 4 atomes de carbone, alcényle comportant jusqu'à 4 atomes de carbone, cycloalkyle comportant 3 - 5 atomes de carbone ou acétyle,
R¹ est un hydrogène, un groupe alkyle comportant 1 - 4 atomes de carbone ou un groupe cycloalkyle comportant 3 - 5 atomes de carbone,
R² est un groupe alkyle substitué de formule 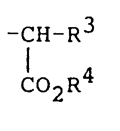 dans laquelle
R³ est un groupe de formule
- (CH₂)ₙ-alkyle(en C₁-c₄),
- (CH₂)ₙ-2-indole,
- (CH₂)ₙ-3-indole, ou
- (CH₂)ₙ-phényle
dans lesquelles n est égal à 0 - 4, et le groupe phényle est non substitué ou mono- ou di-substitué par des substituants X qui sont tels que définis ci-dessus, et
R⁴ est un hydrogène ou un groupe alkyle comportant 1 - 4 atomes de carbone, ou bien le radical
R² est un groupe acyle de formule 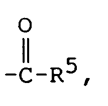 dans laquelle le radical
R⁵ est l'un des groupes suivants :
a) 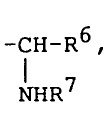 dans lequel
R⁶ est un groupe (CH₂)ₙ-2-indole ou (CH₂)ₙ-3-indole , où n est égal à 0 - 4, et
R⁷ est un hydrogène ou un groupe de formule COOR⁸, ou 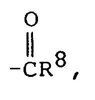 dans laquelle
R⁸ est un groupe alkyle comportant 1 - 4 atomes de carbone,
b) -(CH₂)ₘSCH₂NHCOCH₃, dans lequel m est égal à 1 - 4
c) 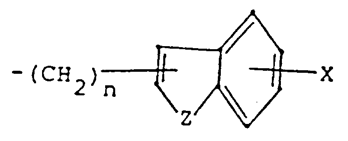
dans lequel
Z est O, S ou NR, où R est tel que défini ci-dessus,
le groupe -(CH₂)ₙ- est fixé sur la position 2 ou sur la position 3,
n est égal à 0 - 4, et
X est tel que défini ci-dessus,
d) 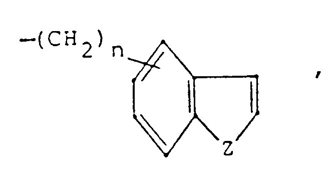
dans lequel
le groupe -(CH₂)ₙ- est fixé en position 4 ou en position 5, et
Z et n sont tels que définis en c) ci-dessus;
e) -(CH₂)ₘCO₂CH₂phényle,
dans lequel
m est égal à 1 - 4,
f) -CHOHC₆H₅;
g) 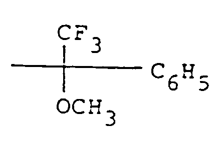 ou
h) un noyau pyrazine, qui est non substitué ou mono-substitué, dans lequel les substituants peuvent être Cl, COOR⁸, CN ou NO₂, où R⁸ est tel que défini en a) ci-dessus;
et les isomères optiques des composés de formule I et les sels ou sels d'ammonium quaternaire des composés de formule I ou II.

2. Composés de formule I ou II selon la revendication 1, formules dans lesquelles
X est un groupe fluoro ou chloro,
Y a la même signification que X,
q et r sont tels que définis ci-dessus,
R est un hydrogène ou un groupe alkyle comportant 1-4 atomes de carbone
R¹ est un hydrogène
R² est un groupe alkyle substitué de formule 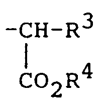 dans lequel
R³ est un groupe de formule
- (CH₂)ₙ-2-indole,
- (CH₂)ₙ-3-indole, ou
- (CH₂)ₙ-phényle
dans lesquelles n est égal à 1, et le groupe phényle est non substitué ou mono- ou di-substitué par des substituants X qui sont tels que définis ci-dessus, et
R⁴ est un groupe alkyle comportant 1 - 4 atomes de carbone, ou bien le radical
R² est un groupe acyle de formule 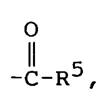 dans laquelle le radical
R⁵ est l'un des groupes suivants :
a) 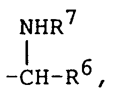
dans lequel
R⁶ est un groupe -CH₂-2-indole ou -CH₂-3-indole, et
R⁷ est tel que défini ci-dessus,
c) 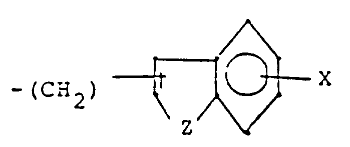
dans lequel Z et X sont tels que définis ci-dessus,
d) 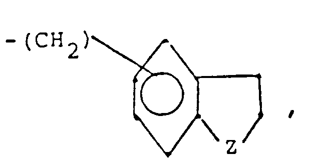 dans lequel Z est tel que défini en c) ci-dessus et le groupe -CH₂- est fixé sur la position 4 ou la position 5, ou
f) -CHOHC₆H₅.

3. Composés de formule I ou II selon la revendication 1, qui sont l'une des substances suivantes :
la 1-méthyl-2-(2'-indolecarbonyl)aminométhyl-5-(2'-fluorophényl)-2,3-dihydro-1H-1,4-benzodiazépine,
la 1-méthyl-2-(4-thianaphtèneméthylcarbonyl)aminométhyl-5-(2'-fluorophényl)-2,3-dihydro-1H-1,4-benzodiazépine,
la 1-méthyl-1-(2-L-hydroxy-2-phénylacétyl)aminométhyl-5-(2'-fluorophényl)-2,3-dihydro-1H-1,4-benzodiazépine,
la 1-méthyl-2-(1H-indole-3-yl)méthylcarbonylaminométhyl-5-(2'-fluorophényl)-2,3-dihydro-1H-1,4-benzodiazépine,
la 1-méthyl-2-[1-(S)-1-méthoxycarbonyl-2-phényléthylamino]-méthyl-5-(2'-fluorophényl)-2,3-dihydro-1H-1,4-benzodiazépine,
la 1-méthyl-2-[2-((1,1-diméthyléthoxy)carbonyl)amino-3-(1H-indole-3-yl)propanoyl]aminométhyl-5-(2'-fluorophényl)-2,3-dihydro-1H-1,4-benzodiazépine,
la 1-méthyl-2-[2-amino-3-(1H-indole-3-yl)propanoyl]aminométhyl-5-(2'-fluorophényl)-2,3-dihydro-1H-1,4-benzodiazépine,
la 1-méthyl-2-(2-méthoxy-2-trifluorométhyl-2-phénylacétyl)aminométhyl-5-(2'-fluorophényl)-2,3-dihydro-1H-1,4-benzodiazépine,
la 1-méthyl-2-[2-(S)-((1,1-diméthyléthoxy)carbonyl)amino-3-acétamidométhylmercaptopropanoyl]aminométhyl-5-(2'-fluorophényl)-2,3-dihydro-1H-1,4-benzodiazépine,
la 1-méthyl-2-benzylsuccinoylaminométhyl-5-(2'-fluorophényl)-2,3-dihydro-1H-1,4-benzodiazépine,
la 1-méthyl-2-(acétamidométhylmercaptoacétyl)aminométhyl-5-(2'-fluorophényl)-2,3-dihydro-1H-1,4-benzodiazépine,
la 1-méthyl-2-(4-thianaphtèneméthylcarbonyl)aminométhyl-5-(2'-fluorophényl)-2,3,4,5-tétrahydro-1H-1,4-benzodiazépine, la 1-méthyl-2-(2-L-hydroxy-2-phénylacétyl)aminométhyl-5-(2'⁻fluorophényl)-2,3,4,5-tétrahydro-1H-1,4-benzodiazépine, ou
la 1-méthyl-2-(1H-indole-3-yl)méthylcarbonylaminométhyl-5-(2'-fluorophényl)-2,3,4,5-tétrahydro-1H-1,4-benzodiazépine.

4. Procédé de préparation de aminométhyl-1,4-benzodiazépines substituées en position 2, répondant à la formule I 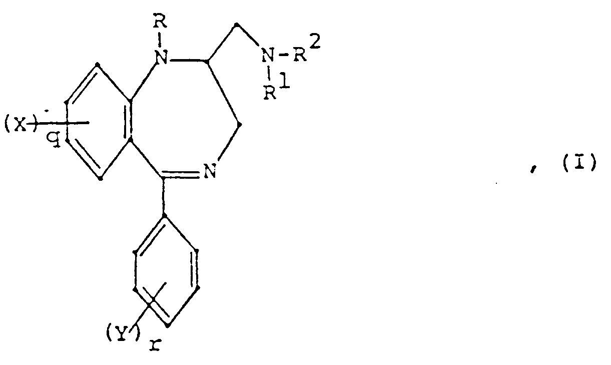 selon la revendication 1,
dans laquelle
X, Y, q, r, R, R¹ et R² sont tels que définis dans la revendication 1,
dans lequel on soumet un composé de formule III 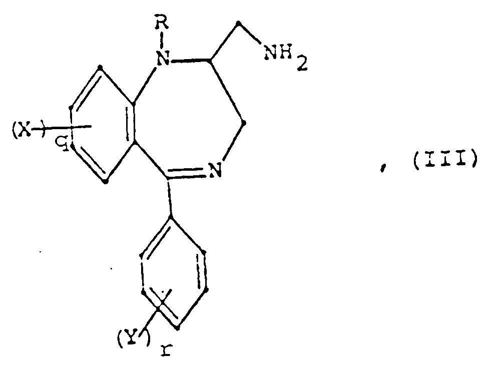 ou un sel d'addition avec un acide du composé de formule III,
formule dans laquelle X, Y, q, r et R sont tels que définis dans la formule I,
à une acylation avec un acide carboxylique de formule R⁵-COOH,
dans laquelle
R⁵ est tel que défini dans la formule I,
ou un dérivé dudit acide carboxylique,
pour obtenir un composé de formule Ia 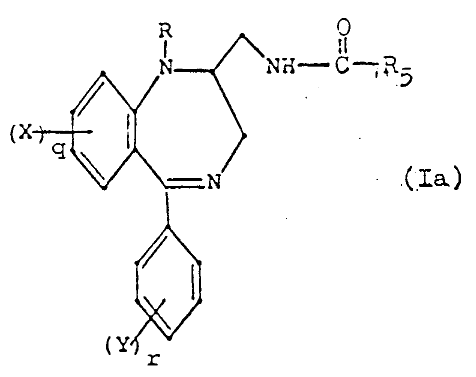 composés de formule Ia qui sont éventuellement alkylés par remplacement de l'atome d'hydrogène lié à l'atome d'azote du groupe amide d'acide par un groupe alkyle comportant 1 - 4 atomes de carbone ou un groupe cycloalkyle comportant 3 - 5 atomes de carbone,
ou
on fait réagir un composé de formule IV 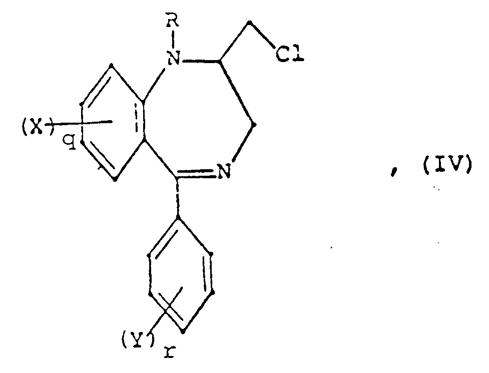 dans laquelle
X, Y, q, r et R sont tels que définis dans la formule I, avec une amine de formule 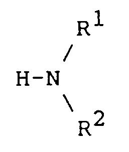 dans laquelle R¹ et R² sont tels que définis dans la formule I, à des températures allant de 0°C au point d'ébullition de l'amine, en présence d'un agent à liaison acide, ou allant de 0°C au point d'ébullition du solvant, en présence d'un solvant aprotique inerte, pour donner un composé de formule I, et on isole les composés amino de formule Ia ou I, ou leurs sels d'addition avec un acide, ou bien éventuellement on transforme les composés de formule Ia ou I en sels d'ammonium quaternaire.

5. Procédé selon la revendication 4, caractérisé en ce que la réaction d'acylation est réalisée dans un solvant aprotique qui est choisi dans le groupe constitué par le N,N-diméthylformamide, le chloroforme, le chlorure de méthylène, le tétrahydrofuranne, le dioxane, le toluène et le chlorobenzène, à une température allant de -30°C au point d'ébullition du solvant, éventuellement en présence d'un réactif de couplage convenable, qui est choisi dans le groupe constitué par le dicyclohexylcarbodiimide, le chlorhydrate de 1-éthyl-3-(3-diméthylaminopropyl)carbodiimide et le carbonyldiimidazole.

6. Procédé selon la revendication 5, dans lequel l'acylation emploie un halogénure d'acide carboxlique ou un anhydride d'acide carboxylique et l'acylation est en outre réalisée en présence d'un agent à liaison acide.

7. Procédé selon la revendication 6, dans lequel l'agent à liaison acide est choisi dans le groupe constitué par une amine tertiaire, un hydroxyde de métal alcalin et un carbonate de métal alcalin.

8. Procédé selon la revendication 7, dans lequel l'agent à liaison acide est choisi dans le groupe constitué par la triéthylamine, la pyridine, la 4-diméthylaminopyridine, l'hydroxyde de sodium et le carbonate de potassium.

9. Procédé de préparation de composés de formule II 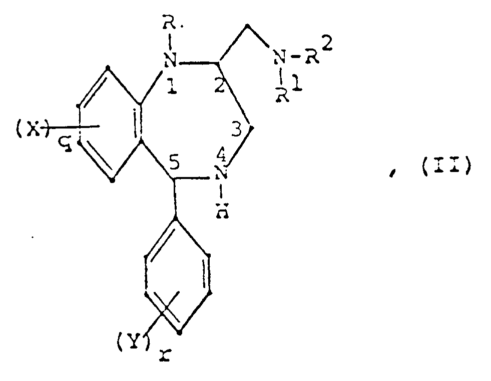 selon la revendication 1,
dans laquelle X, Y, q, r, R, R¹ et R² sont tels que définis dans la revendication 1,
dans lequel on dissout un composé de formule I 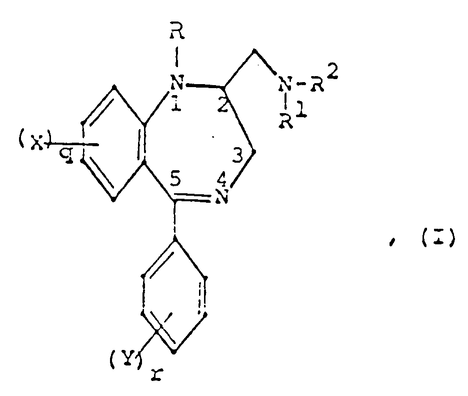 dans un solvant convenable, on refroidit la solution à une température dans la gamme de -30°C à +15°C et on traite le mélange avec un agent réducteur, et on agite jusqu'à ce que la réaction de réduction soit complète, et on verse le mélange réactionnel dans de l'eau et on extrait le composé de formule II et on le lave,
et dans lequel on isole le composé amino libre de formule II ou son sel, ou bien on transforme éventuellement le composé amino libre de formule II en un sel d'ammonium quaternaire.

10. Procédé selon la revendication 9, dans lequel le solvant utilisé dans la réaction de réduction est l'acide acétique glacial, le méthanol ou l'éthanol, et l'agent réducteur est le cyanoborohydrure de sodium, le borohydrure de sodium ou le borohydrure de lithium.

11. Composition pharmaceutique qui est un inhibiteur de la fonction des cholécystokinines chez le mammifère, caractérisée en ce qu'elle comprend, commme ingrédient actif, une quantité pharmaceutiquement efficace d'un ou plusieurs dérivés aminométhyl-1,4-benzodiazépines substitués en position 2 selon la revendication 1, les isomères optiques de ces dérivés ou les sels pharmaceutiquement acceptables ou sels d'ammonium quaternaire desdits composés et, éventuellement, un véhicule pharmaceutiquement acceptable.

12. Composition pharmaceutique selon la revendication 11, comprenant, en outre, un adjuvant.

13. Composition pharmaceutique selon la revendication 11 ou 12, qui comprend, comme ingrédient actif, un composé selon la revendication 3 ou un sel pharmaceutiquement acceptable dudit composé.

14. Composition pharmaceutique selon l'une quelconque des revendications 11 - 13, caractérisée en ce qu elle contient les composés selon la revendication 1 en une quantité telle que la composition peut être utiliser pour l'administration de 0,05 mg dudit composé par kg de poids corporel à 100 mg de ladite substance par kg de poids corporel, en une seule prise ou en plusieurs prises.

15. Composition pharmaceutique selon la revendication 14, caractérisée en ce qu'elle contient le composé pharmaceutiquement efficace en une quantité telle que l'on peut administrer ledit composé en une quantité de 0,5 mg/kg de poids corporel à 20 mg/kg de poids corporel.

16. Composition pharmaceutique selon l'une quelconque des revendications 11 - 15, caractérisée en ce qu'il s'agit d'une composition pharmaceutique pour traiter les êtres humains.
